(19)

Europäisches
Patentamt
European
Patent Office
Office européen
des brevets

(11) **EP 2 565 202 A1**

(12) **EUROPEAN PATENT APPLICATION**
published in accordance with Art. 153(4) EPC

(43) Date of publication:
06.03.2013 Bulletin 2013/10

(21) Application number: 11775142.0

(22) Date of filing: 28.04.2011

(51) Int Cl.:
*C07K 7/06* (2006.01)  *C07K 7/08* (2006.01)
*C07K 14/575* (2006.01)  *C07K 19/00* (2006.01)
*A61K 38/22* (2006.01)  *A61P 5/00* (2006.01)

(86) International application number:
**PCT/JP2011/060446**

(87) International publication number:
**WO 2011/136361 (03.11.2011 Gazette 2011/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 30.04.2010 JP 2010105367

(71) Applicant: **Sanwa Kagaku Kenkyusho Co., Ltd
Nagoya-shi, Aichi 461-8631 (JP)**

(72) Inventors:
• **OKAMOTO, Masayuki
Nagoya-shi
Aichi 461-8631 (JP)**
• **OKAMOTO, Ryuhji
Nagoya-shi
Aichi 461-8631 (JP)**
• **SHIGEMORI, Tomohiro
Kyoto-shi
Kyoto 605-0042 (JP)**

• **MURASE, Takayo
Nagoya-shi
Aichi 461-8631 (JP)**
• **MIYACHI, Atsushi
Nagoya-shi
Aichi 461-8631 (JP)**
• **TAKEUCHI, Mitsuaki
Nagoya-shi
Aichi 461-8631 (JP)**
• **TAMURA, Miyuki
Nagoya-shi
Aichi 461-8631 (JP)**
• **KINOSHITA, Hiroshi
Nagoya-shi
Aichi 461-8631 (JP)**

(74) Representative: **Grünecker, Kinkeldey,
Stockmair & Schwanhäusser
Leopoldstrasse 4
80802 München (DE)**

(54) **PEPTIDE FOR IMPROVING IN VIVO STABILITY OF PHYSIOLOGICALLY ACTIVE SUBSTANCE OR THE LIKE AND PHYSIOLOGICALLY ACTIVE SUBSTANCE WITH IMPROVED IN VIVO STABILITY**

(57)     The present invention aims at providing a peptide fragment capable of improving biostability of a bioactive substance while maintaining the activity of the bioactive substance, and a bioactive substance to which the peptide fragment is added. The present invention relates to a partial peptide of a GA module having 5 to 25 amino acids, including a partial sequence of a GA module (SEQ ID NO: 1) and the amino acid sequence Ile-Asp-Glu-Ile-Leu (SEQ ID NO: 2), and a bioactive complex in which the partial peptide of the GA module is bound to a bioactive substance. The bioactive substance includes GLP-1, GLP-2, GIP, VIP, somatostatin, amylin, ghrelin, derivatives thereof, and the like.

**EP 2 565 202 A1**

SEQUENCE LISTING

<110> SANWA KAGAKU KENKYUSHO CO.,LTD.

<120> Peptides to improve stability in vivo of bioactive peptides and proteins

<130> PCT-SKK1001

<160> 69

<210> 1
<211> 46
<212> PRT
<213> Streptococcus

<220>
<223> GAm(1-46)

<400> 1

Leu Ala Glu Ala Lys Val Leu Ala Asn Arg Glu Leu Asp Lys Tyr Gly
1               5                   10                  15

Val Ser Asp Tyr Tyr Lys Asn Leu Ile Asn Asn Ala Lys Thr Val Glu
            20                  25                  30

Gly Val Lys Ala Leu Ile Asp Glu Ile Leu Ala Ala Leu Pro
            35                  40                  45

<210> 2
<211> 5
<212> PRT
<213> Artificial Sequence

<220>
<223> GAm(38-42)

<400> 2

Ile Asp Glu Ile Leu
1               5

<210> 3
<211> 7
<212> PRT
<213> Artificial Sequence

<220>
<223> GAm(38-44)

<400> 3

Ile Asp Glu Ile Leu Ala Ala
1               5

<210> 4
<211> 8
<212> PRT

```
<213>   Artificial Sequence

<220>
<223>   GAm(37-44)

<400>   4

Leu Ile Asp Glu Ile Leu Ala Ala
1                   5


<210>   5
<211>   10
<212>   PRT
<213>   Artificial Sequence

<220>
<223>   GAm(35-44)

<400>   5

Lys Ala Leu Ile Asp Glu Ile Leu Ala Ala
1                   5                   10


<210>   6
<211>   11
<212>   PRT
<213>   Artificial Sequence

<220>
<223>   GAm(34-44)

<400>   6

Val Lys Ala Leu Ile Asp Glu Ile Leu Ala Ala
1                   5                   10


<210>   7
<211>   11
<212>   PRT
<213>   Artificial Sequence

<220>
<223>   GAm(33-43)

<400>   7

Gly Val Lys Ala Leu Ile Asp Glu Ile Leu Ala
1                   5                   10


<210>   8
<211>   12
<212>   PRT
<213>   Artificial Sequence

<220>
<223>   GAm(33-44)

<400>   8

Gly Val Lys Ala Leu Ile Asp Glu Ile Leu Ala Ala
1                   5                   10
```

```
<210>  9
<211>  13
<212>  PRT
<213>  Artificial Sequence

<220>
<223>  GAm(32-44)

<400>  9

Glu Gly Val Lys Ala Leu Ile Asp Glu Ile Leu Ala Ala
1               5                   10


<210>  10
<211>  14
<212>  PRT
<213>  Artificial Sequence

<220>
<223>  GAm(31-44)

<400>  10

Val Glu Gly Val Lys Ala Leu Ile Asp Glu Ile Leu Ala Ala
1               5                   10


<210>  11
<211>  13
<212>  PRT
<213>  Artificial Sequence

<220>
<223>  GAm(30-42)

<400>  11

Thr Val Glu Gly Val Lys Ala Leu Ile Asp Glu Ile Leu
1                   5               10


<210>  12
<211>  15
<212>  PRT
<213>  Artificial Sequence

<220>
<223>  GAm(28-42)

<400>  12
Ala Lys Thr Val Glu Gly Val Lys Ala Leu Ile Asp Glu Ile Leu
1                   5                   10                  15


<210>  13
<211>  17
<212>  PRT
<213>  Artificial Sequence

<220>
<223>  GAm(28-44)
```

<400> 13

Ala Lys Thr Val Glu Gly Val Lys Ala Leu Ile Asp Glu Ile Leu Ala
1               5                   10                  15

Ala


<210> 14
<211> 20
<212> PRT
<213> Artificial Sequence

<220>
<223> GAm(27-46)

<400> 14

Asn Ala Lys Thr Val Glu Gly Val Lys Ala Leu Ile Asp Glu Ile Leu
1               5                   10                  15

Ala Ala Leu Pro
            20


<210> 15
<211> 22
<212> PRT
<213> Artificial Sequence

<220>
<223> GAm(25-46)

<400> 15

Ile Asn Asn Ala Lys Thr Val Glu Gly Val Lys Ala Leu Ile Asp Glu
1               5                   10                  15

Ile Leu Ala Ala Leu Pro
            20


<210> 16
<211> 25
<212> PRT
<213> Artificial Sequence

<220>
<223> GAm(22-46)

<400> 16

Lys Asn Leu Ile Asn Asn Ala Lys Thr Val Glu Gly Val Lys Ala Leu
1               5                   10                  15

Ile Asp Glu Ile Leu Ala Ala Leu Pro
            20                  25


<210> 17

```
<211>  30
<212>  PRT
<213>  Artificial Sequence

<220>
<223>  GAm(17-46)

<400>  17

Val Ser Asp Tyr Tyr Lys Asn Leu Ile Asn Asn Ala Lys Thr Val Glu
1               5                   10                  15

Gly Val Lys Ala Leu Ile Asp Glu Ile Leu Ala Ala Leu Pro
            20                  25                  30


<210>  18
<211>  26
<212>  PRT
<213>  Artificial Sequence

<220>
<223>  GAm(21-46)

<400>  18

Tyr Lys Asn Leu Ile Asn Asn Ala Lys Thr Val Glu Gly Val Lys Ala
1               5                   10                  15

Leu Ile Asp Glu Ile Leu Ala Ala Leu Pro
            20                  25


<210>  19
<211>  13
<212>  PRT
<213>  Artificial Sequence

<220>
<223>  GAm(28-40)

<400>  19

Ala Lys Thr Val Glu Gly Val Lys Ala Leu Ile Asp Glu
1                   5                   10


<210>  20
<211>  10
<212>  PRT
<213>  Artificial Sequence

<220>
<223>  GAm(28-37)

<400>  20

Ala Lys Thr Val Glu Gly Val Lys Ala Leu
1                   5                   10


<210>  21
<211>  6
```

28

```
<212>  PRT
<213>  Artificial Sequence

<220>
<223>  GAm(39-44)

<400>  21

Asp Glu Ile Leu Ala Ala
1               5


<210>  22
<211>  4
<212>  PRT
<213>  Artificial Sequence

<220>
<223>  GAm(38-41)

<400>  22

Ile Asp Glu Ile
1


<210>  23
<211>  3
<212>  PRT
<213>  Artificial Sequence

<220>
<223>  GAm(38-40)

<400>  23

Ile Asp Glu
1


<210>  24
<211>  30
<212>  PRT
<213>  Homo sapiens

<220>
<223>  GLP-1(7-36)

<400>  24

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30


<210>  25
<211>  29
<212>  PRT
<213>  Artificial Sequence

<220>
<223>  GLP-1(7-35)
```

<400> 25

```
His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly
            20                  25
```

<210> 26
<211> 30
<212> PRT
<213> Artificial Sequence

<220>
<223> 8S-GLP-1(7-36)

<400> 26

```
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30
```

<210> 27
<211> 39
<212> PRT
<213> Gila monster

<220>
<223> Exendin-4(1-39)

<400> 27

```
His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
            35
```

<210> 28
<211> 34
<212> PRT
<213> Artificial Sequence

<220>
<223> 8S-GLP-1(7-35) + GAm(38-42)

<400> 28

```
His Ser Glu Gly Thr Phe Thr Ser Asp Val Ser Ser Tyr Leu Glu Gly
1               5                   10                  15
```

```
Gln Ala Ala Lys Glu Phe Ile Ala Trp Leu Val Lys Gly Ile Asp Glu
            20                  25                  30


Ile Leu



<210>   29
<211>   36
<212>   PRT
<213>   Artificial Sequence

<220>
<223>   8S-GLP-1(7-35) + GAm(38-44)

<400>   29

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Ile Asp Glu
            20                  25                  30


Ile Leu Ala Ala
            35


<210>   30
<211>   37
<212>   PRT
<213>   Artificial Sequence

<220>
<223>   8S-GLP-1(7-35) + GAm(37-44)

<400>   30

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Leu Ile Asp
            20                  25                  30


Glu Ile Leu Ala Ala
            35


<210>   31
<211>   39
<212>   PRT
<213>   Artificial Sequence

<220>
<223>   8S-GLP-1(7-35) + GAm(35-44)

<400>   31

His Ser Glu Gly Thr Phe Thr Ser Asp Val Ser Ser Tyr Leu Glu Gly
1               5                   10                  15
```

```
Gln Ala Ala Lys Glu Phe Ile Ala Trp Leu Val Lys Gly Lys Ala Leu
            20                  25                  30


Ile Asp Glu Ile Leu Ala Ala
            35



<210>  32
<211>  34
<212>  PRT
<213>  Artificial Sequence

<220>
<223>  GLP-1(7-35) + GAm(38-42)

<400>  32

His Ala Glu Gly Thr Phe Thr Ser Asp Val Ser Ser Tyr Leu Glu Gly
1                5                  10                  15


Gln Ala Ala Lys Glu Phe Ile Ala Trp Leu Val Lys Gly Ile Asp Glu
            20                  25                  30


Ile Leu



<210>  33
<211>  39
<212>  PRT
<213>  Artificial Sequence

<220>
<223>  GLP-1(7-35) + GAm(35-44)

<400>  33

His Ala Glu Gly Thr Phe Thr Ser Asp Val Ser Ser Tyr Leu Glu Gly
1                5                  10                  15


Gln Ala Ala Lys Glu Phe Ile Ala Trp Leu Val Lys Gly Lys Ala Leu
            20                  25                  30


Ile Asp Glu Ile Leu Ala Ala
            35



<210>  34
<211>  40
<212>  PRT
<213>  Artificial Sequence

<220>
<223>  GLP-1(7-35) + GAm(33-43)

<400>  34

His Ala Glu Gly Thr Phe Thr Ser Asp Val Ser Ser Tyr Leu Glu Gly
1                5                  10                  15
```

```
Gln Ala Ala Lys Glu Phe Ile Ala Trp Leu Val Lys Gly Gly Val Lys
          20                  25                  30

Ala Leu Ile Asp Glu Ile Leu Ala
          35                  40


<210>  35
<211>  41
<212>  PRT
<213>  Artificial Sequence

<220>
<223>  GLP-1(7-35) + GAm(33-44)

<400>  35

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Gly Val Lys
          20                  25                  30


Ala Leu Ile Asp Glu Ile Leu Ala Ala
          35                  40


<210>  36
<211>  41
<212>  PRT
<213>  Artificial Sequence

<220>
<223>  8S-GLP-1(7-35) + GAm(33-44)

<400>  36

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Gly Val Lys
          20                  25                  30


Ala Leu Ile Asp Glu Ile Leu Ala Ala
          35                  40


<210>  37
<211>  42
<212>  PRT
<213>  Artificial Sequence

<220>
<223>  8S-GLP-1(7-35) + GAm(32-44)

<400>  37

His Ser Glu Gly Thr Phe Thr Ser Asp Val Ser Ser Tyr Leu Glu Gly
```

```
                  1                 5                   10                  15


                  Gln Ala Ala Lys Glu Phe Ile Ala Trp Leu Val Lys Gly Glu Gly Val
                         20              25              30


                  Lys Ala Leu Ile Asp Glu Ile Leu Ala Ala
                         35              40


                  <210>  38
                  <211>  43
                  <212>  PRT
                  <213>  Artificial Sequence

                  <220>
                  <223>  8S-GLP-1(7-35) + GAm(31-44)

                  <400>  38

                  His Ser Glu Gly Thr Phe Thr Ser Asp Val Ser Ser Tyr Leu Glu Gly
                  1               5                   10                  15


                  Gln Ala Ala Lys Glu Phe Ile Ala Trp Leu Val Lys Gly Val Glu Gly
                         20              25              30


                  Val Lys Ala Leu Ile Asp Glu Ile Leu Ala Ala
                         35              40


                  <210>  39
                  <211>  42
                  <212>  PRT
                  <213>  Artificial Sequence

                  <220>
                  <223>  8S-GLP-1(7-35) + GAm(30-42)

                  <400>  39

                  His Ser Glu Gly Thr Phe Thr Ser Asp Val Ser Ser Tyr Leu Glu Gly
                  1               5                   10                  15


                  Gln Ala Ala Lys Glu Phe Ile Ala Trp Leu Val Lys Gly Thr Val Glu
                         20              25              30


                  Gly Val Lys Ala Leu Ile Asp Glu Ile Leu
                         35              40


                  <210>  40
                  <211>  44
                  <212>  PRT
                  <213>  Artificial Sequence

                  <220>
                  <223>  8S-GLP-1(7-35) + GAm(28-42)

                  <400>  40
```

```
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Ala Lys Thr
            20                  25                  30


Val Glu Gly Val Lys Ala Leu Ile Asp Glu Ile Leu
        35                  40
```

```
<210>  41
<211>  46
<212>  PRT
<213>  Artificial Sequence

<220>
<223>  8S-GLP-1(7-35) + GAm(28-44)

<400>  41
```

```
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Ala Lys Thr
            20                  25                  30


Val Glu Gly Val Lys Ala Leu Ile Asp Glu Ile Leu Ala Ala
        35                  40                  45
```

```
<210>  42
<211>  49
<212>  PRT
<213>  Artificial Sequence

<220>
<223>  8S-GLP-1(7-35) + GAm(27-46)

<400>  42
```

```
His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Asn Ala Lys
            20                  25                  30


Thr Val Glu Gly Val Lys Ala Leu Ile Asp Glu Ile Leu Ala Ala Leu
        35                  40                  45


Pro
```

```
<210>  43
<211>  51
<212>  PRT
<213>  Artificial Sequence
```

<220>
<223>   8S-GLP-1(7-35) + GAm(25-46)

<400>   43

His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Ile Asn Asn
            20                  25                  30

Ala Lys Thr Val Glu Gly Val Lys Ala Leu Ile Asp Glu Ile Leu Ala
        35                  40                  45

Ala Leu Pro
    50


<210>   44
<211>   54
<212>   PRT
<213>   Artificial Sequence

<220>
<223>   8S-GLP-1(7-35) + GAm(22-46)

<400>   44

His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Lys Asn Leu
            20                  25                  30

Ile Asn Asn Ala Lys Thr Val Glu Gly Val Lys Ala Leu Ile Asp Glu
        35                  40                      45

Ile Leu Ala Ala Leu Pro
    50


<210>   45
<211>   41
<212>   PRT
<213>   Artificial Sequence

<220>
<223>   GLP-1(7-35) + linker(GP) + GAm(35-44)

<400>   45

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Gly Pro Lys
            20                  25                  30

```
Ala Leu Ile Asp Glu Ile Leu Ala Ala
        35                  40


<210>  46
<211>  42
<212>  PRT
<213>  Artificial Sequence


<220>
<223>  GLP-1(7-35) + linker(GP) + GAm(34-44)


<400>  46


His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Gly Pro Val
            20                  25                  30


Lys Ala Leu Ile Asp Glu Ile Leu Ala Ala
        35                  40


<210>  47
<211>  43
<212>  PRT
<213>  Artificial Sequence


<220>
<223>  GLP-1(7-35) + linker(GPS) + GAm(34-44)


<400>  47


His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Gly Pro Ser
            20                  25                  30


Val Lys Ala Leu Ile Asp Glu Ile Leu Ala Ala
            35                  40


<210>  48
<211>  44
<212>  PRT
<213>  Artificial Sequence


<220>
<223>  GLP-1(7-35) + linker(GPS) + GAm(33-44)


<400>  48


His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Gly Pro Ser
            20                  25                  30
```

Gly Val Lys Ala Leu Ile Asp Glu Ile Leu Ala Ala
        35                  40


<210>  49
<211>  75
<212>  PRT
<213>  Artificial Sequence

<220>
<223>  8S-GLP-1(7-35) + GAm(1-46)

<400>  49

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Leu Ala Glu
            20                  25                  30


Ala Lys Val Leu Ala Asn Arg Glu Leu Asp Lys Tyr Gly Val Ser Asp
            35                  40                  45


Tyr Tyr Lys Asn Leu Ile Asn Asn Ala Lys Thr Val Glu Gly Val Lys
        50                  55                  60


Ala Leu Ile Asp Glu Ile Leu Ala Ala Leu Pro
65                  70                  75


<210>  50
<211>  59
<212>  PRT
<213>  Artificial Sequence

<220>
<223>  8S-GLP-1(7-35) + GAm(17-46)

<400>  50

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Val Ser Asp
            20                  25                  30


Tyr Tyr Lys Asn Leu Ile Asn Asn Ala Lys Thr Val Glu Gly Val Lys
            35                  40                  45


Ala Leu Ile Asp Glu Ile Leu Ala Ala Leu Pro
        50                  55


<210>  51
<211>  55
<212>  PRT
<213>  Artificial Sequence

```
<220>
<223>   8S-GLP-1(7-35) + GAm(21-46)

<400>   51

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Tyr Lys Asn
            20                  25                  30


Leu Ile Asn Asn Ala Lys Thr Val Glu Gly Val Lys Ala Leu Ile Asp
        35                  40                  45


Glu Ile Leu Ala Ala Leu Pro
    50                  55


<210>   52
<211>   42
<212>   PRT
<213>   Artificial Sequence

<220>
<223>   8S-GLP-1(7-35) + GAm(28-40)

<400>   52

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Ala Lys Thr
            20                  25                  30


Val Glu Gly Val Lys Ala Leu Ile Asp Glu
        35                  40


<210>   53
<211>   39
<212>   PRT
<213>   Artificial Sequence

<220>
<223>   8S-GLP-1(7-35) + GAm(28-37)

<400>   53

His Ser Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Ala Lys Thr
            20                  25                  30


Val Glu Gly Val Lys Ala Leu
        35
```

```
<210>   54
<211>   35
<212>   PRT
<213>   Artificial Sequence

<220>
<223>   8S-GLP-1(7-35) + GAm(39-44)

<400>   54

His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Asp Glu Ile
            20                  25                  30

Leu Ala Ala
        35


<210>   55
<211>   33
<212>   PRT
<213>   Artificial Sequence

<220>
<223>   GLP-1(7-35) + GAm(38-41)

<400>   55

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Ile Asp Glu
            20                  25                  30

Ile


<210>   56
<211>   32
<212>   PRT
<213>   Artificial Sequence

<220>
<223>   GLP-1(7-35) + GAm(38-40)

<400>   56

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Ile Asp Glu
            20                  25                  30


<210>   57
<211>   33
```

EP 2 565 202 A1

<212> PRT
<213> Artificial Sequence

<220>
<223> 8G-GLP-1(7-35) + RPSS

<400> 57

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Pro Ser
            20                  25                  30

Ser


<210> 58
<211> 39
<212> PRT
<213> Artificial Sequence

<220>
<223> 8S,22,23E-GLP-1(7-35) + GPSSGAPPPS

<400> 58

His Ser Glu Gly Thr Phe Thr Ser Asp Val Ser Ser Tyr Leu Glu Glu
1               5                   10                  15

Glu Ala Ala Lys Glu Phe Ile Ala Trp Leu Val Lys Gly Gly Pro Ser
            20                  25                  30

Ser Gly Ara Pro Pro Pro Ser
            35


<210> 59
<211> 28
<212> PRT
<213> Homo sapiens

<220>
<223> VIP

<400> 59

His Ser Asp Ala Val Phe Thr Asp Asn Tyr Thr Arg Leu Arg Lys Gln
1               5                   10                  15

Met Ala Val Lys Lys Tyr leu Asn Ser Ile Leu Asn
            20                  25


<210> 60
<211> 36
<212> PRT
<213> Artificial Sequence

41

```
<220>
<223>  VIP + GAm(37-44)

<400>  60

His Ser Asp Ala Val Phe Thr Asp Asn Tyr Thr Arg Leu Arg Lys Gln
1               5                   10                  15


Met Ala Val lys Lys Tyr leu Asn Ser Ile Leu Asn Leu Ile Asp Glu
            20                  25                  30


Ile Leu Ala Ala
        35



<210>  61
<211>  41
<212>  PRT
<213>  Artificial Sequence

<220>
<223>  VIP + GAm(32-44)

<400>  61

His Ser Asp Ala Val Phe Thr Asp Asn Tyr Thr Arg Leu Arg Lys Gln
1               5                   10                  15


Met Ala Val lys Lys Tyr leu Asn Ser Ile Leu Asn Glu Gly Val Lys
            20                  25                  30


Ala Leu Ile Asp Glu Ile Leu Ala Ala
        35                  40



<210>  62
<211>  45
<212>  PRT
<213>  Artificial Sequence

<220>
<223>  VIP + GAm(28-44)

<400>  62

His Ser Asp Ala Val Phe Thr Asp Asn Tyr Thr Arg Leu Arg Lys Gln
1               5                   10                  15


Met Ala Val lys Lys Tyr leu Asn Ser Ile Leu Asn Ala Lys Thr Val
            20                  25                  30


Glu Gly Val Lys Ala Leu Ile Asp Glu Ile Leu Ala Ala
        35                  40                  45



<210>  63
<211>  14
<212>  PRT
<213>  Homo sapiens
```

```
<220>
<223>  SRIF

<220>
<221>  DISULFID
<222>  3..14

<400>  63

Ala Gly Cys Lys Asn Phe Phe Trp Lys Thr Phe Thr Ser Cys
1               5                   10


<210>  64
<211>  31
<212>  PRT
<213>  Artificial Sequence

<220>
<223>  SRIF + GAm(28-44)

<220>
<221>  DISULFID
<222>  3..14

<400>  64

Ala Gly Cys Lys Asn Phe Phe Trp Lys Thr Phe Thr Ser Cys Ala Lys
1               5                   10                  15


Thr Val Glu Gly Val Lys Ala Leu Ile Asp Glu Ile Leu Ala Ala
            20                  25                  30


<210>  65
<211>  31
<212>  PRT
<213>  Artificial Sequence

<220>
<223>  GAm(28-44) + SRIF

<220>
<221>  DISULFID
<222>  20..31

<400>  65

Ala Lys Thr Val Glu Gly Val Lys Ala Leu Ile Asp Glu Ile Leu Ala
1               5                   10                  15


Ala Ala Gly Cys Lys Asn Phe Phe Trp Lys Thr Phe Thr Ser Cys
            20                  25                  30


<210>  66
<211>  37
<212>  PRT
<213>  Homo sapiens

<220>
<223>  Amylin
```

```
<220>
<221>  DISULFID
<222>  2..7

<400>  66

Lys Cys Asn Thr Ala Thr Cys Ala Thr Gln Arg Leu Ala Asn Phe Leu
1               5                  10                  15


Val His Ser Ser Asn Asn Phe Gly Ala Ile Leu Ser Ser Thr Asn Val
            20                  25                  30


Gly Ser Asn Thr Tyr
        35


<210>  67
<211>  54
<212>  PRT
<213>  Artificial Sequence

<220>
<223>  GAm(28-44) + Amylin

<220>
<221>  DISULFID
<222>  19..24

<400>  67

Ala Lys Thr Val Glu Gly Val Lys Ala Leu Ile Asp Glu Ile Leu Ala
1               5                  10                  15


Ala Lys Cys Asn Thr Ala Thr Cys Ala Thr Gln Arg Leu Ala Asn Phe
            20                  25                  30


Leu Val His Ser Ser Asn Asn Phe Gly Ala Ile Leu Ser Ser Thr Asn
        35                  40                  45


Val Gly Ser Asn Thr Tyr
        50


<210>  68
<211>  28
<212>  PRT
<213>  Homo sapiens

<220>
<223>  Ghrelin

<220>
<221>  MOD_RES
<222>  3..3
<223>  modified with n-octanoyl

<400>  68

Gly Ser Ser Phe Leu Ser Pro Glu His Gln Arg Val Gln Gln Arg Lys
```

```
         1                 5                 10                15


         Glu Ser Lys Lys Pro Pro Ala Lys Leu Gln Pro Arg
                     20                 25



         <210>  69
         <211>  45
         <212>  PRT
         <213>  Artificial Sequence

         <220>
         <223>  Ghrelin + GAm(28-44)

         <220>
         <221>  MOD_RES
         <222>  3..3
         <223>  modified with n-octanoyl

         <400>  69

         Gly Ser Ser Phe Leu Ser Pro Glu His Gln Arg Val Gln Gln Arg Lys
         1                 5                 10                15


         Glu Ser Lys Lys Pro Pro Ala Lys Leu Gln Pro Arg Ala Lys Thr Val
                     20                 25                 30


         Glu Gly Val Lys Ala Leu Ile Asp Glu Ile Leu Ala Ala
                     35                 40                 45
```

## Description

### TECHNICAL FIELD

The present invention relates to a peptide for improving biostability of bioactive substances and the like, and a peptide complex in which the peptide is bound to a bioactive substance.

### BACKGROUND ART

Rapid elimination of bioactive substances (such as proteins, peptides, and the like) *in vivo* may sometimes restrict the efficacies of medicine when the substances are applied to the medicine. For example, it is believed that physiological actions of GLP-1 (glucagon like peptide-1) are useful in medicine for diabetes, but the elimination half-life in blood thereof is from 2 to 3 minutes, and therefore a continuous infusion or frequent administration is necessary for obtaining a sufficient efficacy. As one solution thereof, fatty acid-modified GLP-1s are studied (Non-Patent Document 1 and Patent Document 1). That is to say, as a result of modification of GLP-1 with fatty acid thereby imparting an affinity to albumin, which is a blood protein, enzymolysis and renal excretion are avoided, and the improvement of elimination half-life in blood can be attained. When the modification is performed using a fatty acid, a hydrophobic long-chain molecule such as polyethylene glycol (PGE), or a polyether, however, the problems of complicated production steps and decreased production yields arise.

It is known that some kinds of proteins derived from Gram-positive bacteria such as Protein G have a region having a high affinity to albumin, and a peptide of 46 amino acid residues called as a GA module or an ABD (Albumin binding domain) are known as a minimum region having the ability described above (Non-Patent Document 2). In fact, it is known that some kinds of antibody proteins to which the GA module is added have biostability more improved than those of antibody proteins having no GA module (Non-Patent Document 3). Similarly, it is also known that when a peptide segment derived from staphylococcal protein A or streptococcal protein G and bound to serum albumin or immunoglobulin G is bound to a bioactive protein, the half-life in vivo can be prolonged (Patent Document 3). It is known that a peptide composed of a GA module sequence has an albumin binding capacity at the second helix part and the third helix part of the GA module (Patent Document 2 and Non-Patent Document 4).

### Prior Art Technical Documents

### Patent Documents

Patent Document 1 WO1996/029342
Patent Document 2 WO2009/016043
Patent Document 3 WO91/001743

### Non-Patent Documents

Non-Patent Document 1 J Med Chem, 2000, 43 (9), 1664-1669
Non-Patent Document 2 Biochemistry, 1992, 31 (5), 1451-1457
Non-Patent Document 3 Protein Eng Des Sel, 2007, 20 (11), 569-576
Non-Patent Document 4 Protein Eng Des Sel, 2008, 21 (8), 515-527

### SUMMARY OF INVENTION

### Technical Problem

As stated above, it can be expected that when the GA module is added to a bioactive substance, the biostability of the bioactive substance is improved. However, as a result of the present inventors' study, they have found a new problem that if the peptide of the GA module 46 residues is added to the bioactive substance or the like, the activity thereof is remarkably decreased compared with those having no peptide of the GA module 46 residues. The present invention, accordingly, aims at providing a peptide fragment capable of improving the biostability while maintaining the activity of the bioactive substance.

**Solution to Problem**

The present inventors have considered that the remarkable decrease of the activity caused by the addition of the peptide of the GA module 46 residues to the bioactive substance results from a difference in the length of the added amino acid sequence, and have painstakingly studied to find a particularly essential site for improvement of the biostability of the bioactive substance, with a focus on the third helix part in the GA module 46 residues. As a result, they have found a peptide including a partial sequence of the GA module (hereinafter referred to as the "partial peptide of the GA module") capable of significantly avoiding the decrease of the activity of the bioactive substance and improving the biostability thereof, and have completed the present invention.

That is to say, the present inventions have main constituent features as follows:

(1) A partial peptide of a GA module, comprising the amino acid sequence: Ile-Asp-Glu-Ile-Leu and having 5 to 25 amino acids.
(2) The partial peptide according to the item (1) above, wherein the GA module is derived from a streptococcus G148.
(3) The partial peptide according to the item (1) above, which has 5 to 17 amino acids.

(4) The partial peptide according to the item (1) above, which consists of the amino acid sequence depicted by the formula: $Y_{22}$-$Y_{23}$-$Y_{24}$-$Y_{25}$-$Y_{26}$-$Y_{27}$-$Y_{28}$-$Y_{29}$-$Y_{30}$-$Y_{31}$-$Y_{32}$-$Y_{33}$-$Y_{34}$-$Y_{35}$-$Y_{36}$-$Y_{37}$-Ile-Asp-Glu-Ile-Leu-$Y_{43}$-$Y_{44}$-$Y_{45}$-$Y_{46}$ wherein: $Y_{22}$ is Lys or deletion; $Y_{23}$ is Asn or deletion; $Y_{24}$ is Leu or deletion; $Y_{25}$ is Ile or deletion; $Y_{26}$ is Asn or deletion; $Y_{27}$ is Asn or deletion; $Y_{28}$ is Ala or deletion; $Y_{29}$ is Lys or deletion; $Y_{30}$ is Thr or deletion; $Y_{31}$ is Val or deletion; $Y_{32}$ is Glu or deletion; $Y_{33}$ is Gly or deletion; $Y_{34}$ is Val or deletion; $Y_{35}$ is Lys or deletion; $Y_{36}$ is Ala or deletion; $Y_{37}$ is Leu or deletion; $Y_{43}$ is Ala or deletion; $Y_{44}$ is Ala or deletion; $Y_{45}$ is Leu or deletion; and $Y_{46}$ is Pro or deletion, provided that the deletion is limited to a continuous deletion from $Y_{22}$ and/or $Y_{46}$ (including single deletion of $Y_{22}$ or $Y_{46}$, and deletions at two portions of $Y_{22}$ and $Y_{46}$ alone).

(5) The partial peptide according to the item (4) above, which is selected from the group consisting of:

Ile-Asp-Glu-Ile-Leu (SEQ ID NO: 2);
Ile-Asp-Glu-Ile-Leu-Ala-Ala (SEQ ID NO: 3);
Leu-Ile-Asp-Glu-Ile-Leu-Ala-Ala (SEQ ID NO: 4);
Lys-Ala-Leu-Ile-Asp-Glu-Ile-Leu-Ala-Ala (SEQ ID NO: 5);
Val-Lys-Ala-Leu-Ile-Asp-Glu-Ile-Leu-Ala-Ala (SEQ ID NO: 6);
Gly-Val-Lys-Ala-Leu-Ile-Asp-Glu-Ile-Leu-Ala (SEQ ID NO: 7);
Gly-Val-Lys-Ala-Leu-Ile-Asp-Glu-Ile-Leu-Ala-Ala (SEQ ID NO: 8);
Glu-Gly-Val-Lys-Ala-Leu-Ile-Asp-Glu-Ile-Leu-Ala-Ala (SEQ ID NO: 9);
Val-Glu-Gly-Val-Lys-Ala-Leu-Ile-Asp-Glu-Ile-Leu-Ala-Ala (SEQ ID NO: 10);
Thr-Val-Glu-Gly-Val-Lys-Ala-Leu-Ile-Asp-Glu-Ile-Leu (SEQ ID NO: 11);
Ala-Lys-Thr-Val-Glu-Gly-Val-Lys-Ala-Leu-Ile-Asp-Glu-Ile-Leu (SEQ ID NO: 12);
Ala-Lys-Thr-Val-Glu-Gly-Val-Lys-Ala-Leu-Ile-Asp-Glu-Ile-Leu-Ala-Ala (SEQ ID NO: 13);
Asn-Ala-Lys-Thr-Val-Glu-Gly-Val-Lys-Ala-Leu-Ile-Asp-Glu-Ile-Leu-Ala-Ala-Leu-Pro (SEQ ID NO: 14);
Ile-Asn-Asn-Ala-Lys-Thr-Val-Glu-Gly-Val-Lys-Ala-Leu-Ile-Asp-Glu-Ile-Leu-Ala-Ala-Leu-Pro (SEQ ID NO: 15); and
Lys-Asn-Leu-Ile-Asn-Asn-Ala-Lys-Thr-Val-Glu-Gly-Val-Lys-Ala-Leu-Ile-Asp-Glu-Ile-Leu-Ala-Ala-Leu-Pro (SEQ ID NO: 16).

(6) The partial peptide, which starts with one of the amino acid positions 22 to 38 of the GA module depicted in SEQ ID NO: 1 and ends with one of the amino acid positions 42 to 46 thereof.
(7) A bioactive complex comprising the partial peptide according to any one of the items (1) to (6) and a bioactive substance bound to the partial peptide.
(8) The bioactive complex according to the item (7) above, wherein the bioactive substance is a gastrointestinal hormone or a derivative thereof, or Exendin-4 or a derivative thereof.

(9) The bioactive complex according to the item (8) above, wherein the gastrointestinal hormone is selected from the group consisting of GLP-1, GLP-2, GIP, VIP, somatostatin, amylin and ghrelin.
(10) The bioactive complex according to the item (7) above, further comprising a linker through which the partial peptide and the bioactive substance are bound.

(11) The bioactive complex according to the item (9) above, wherein the GLP-1 or the derivative thereof has GLP-1 activity and consists of the amino acid sequence depicted by the formula: His-$X_8$-$X_9$-Gly-Thr-Phe-Thr-Ser-Asp-$X_{16}$-Ser-$X_{18}$-$X_{19}$-$X_{20}$-Glu-$X_{22}$-$X_{23}$-Ala-$X_{25}$-$X_{26}$-$X_{27}$-Phe -Ile-$X_{30}$-Trp-Leu-$X_{33}$-$X_{34}$-$X_{35}$-$X_{36}$-$X_{37}$-$X_{38}$-$X_{39}$-$X_{40}$-$X_{41}$-$X_{42}$-$X_{43}$-$X_{44}$-$X_{45}$
wherein: $X_8$ is Ala, Gly, Ser, Thr, Leu, Ile, Val, Glu, Asp, Lys or Aib; $X_9$ is Glu, Gly, Asp or Lys; $X_{16}$ is Val, Ala, Gly, Ser, Thr, Leu, Ile, Tyr, Glu, Asp, Trp or Lys; $X_{18}$ is Ser, Ala, Arg, Gly, Thr, Leu, Ile, Val, Glu, Asp, Trp or Lys; $X_{19}$ is Tyr, Phe, Trp, Glu, Gln, Asp or Lys; $X_{20}$ is Leu, Met, Ala, Gly, Ser, Thr, Leu, Ile, Val, Glu, Asp, Trp, Tyr or Lys; $X_{22}$ is Gly, Ala, Glu, Ser, Thr, Leu, Ile, Val, Asp, Lys, Arg, Cys or Aib; $X_{23}$ is Gln, Arg, Glu, Asp, Asn or Lys; $X_{25}$ is Ala, Gly, Ser, Thr, Leu, Ile, Val, Glu, Asp or Lys; $X_{26}$ is Lys, Gln, Glu, Asp, His or Arg; $X_{27}$ is Glu, Ala, Asp, Lys or Leu; $X_{30}$ is Ala, Glu, Gly, Ser, Thr, Leu, Ile, Val, Asp, Lys, Gln, Tyr, His or Arg; $X_{33}$ is Val, Gly, Ala, Ser, Thr, Leu, Ile, Glu, Asp or Lys; $X_{34}$ is Lys, Arg, Glu, Asp, His, Ala, Gly or Asn; $X_{35}$ is Gly, Ala, Ser, Thr, Leu, Ile, Val, Glu, Asp, Lys, His, Pro or Aib; $X_{36}$ is Arg, Gly, Lys, Glu, Asp, His or deletion; $X_{37}$ is Gly, Pro, Glu, Lys, Ala, Thr, Ser, Asp, Leu, Ile, Val, His or deletion; $X_{38}$ is Ser, Lys, Arg, Glu, Asp, His or deletion; $X_{39}$ is Ser, Arg, Lys, Glu, Asp, His or deletion; $X_{40}$ is Gly, Glu, Lys, Asp or deletion; $X_{41}$ is Ala, Lys, Phe, Trp, Tyr, Glu, Asp or deletion; $X_{42}$ is Pro, Lys, Glu, Asp or deletion; $X_{43}$ is Pro, Lys, Glu, Asp or deletion; $X_{44}$ is Pro, Lys, Glu, Asp or deletion; and $X_{45}$ is Ser, Lys, Val, Glu, Asp or deletion.

(12) The bioactive complex according to the item (11) above, wherein the GLP-1 or the derivative thereof is selected from the group consisting of: GLP-1 (7-37); [Ser8]-GLP-1 (7-37); [Gly8]-GLP-1 (7-37); [Val8]-GLP-1 (7-37); [Glu22]-GLP-1 (7-37); [Lys22]-GLP-1 (7-37); [Val8, Glu22]-GLP-1 (7-37); [Val8, Lys22]-GLP-1 (7-37); [Gly8, Glu22]-GLP-1 (7-37); [Gly8, Lys22]-GLP-1 (7-37); [Val8, Glu30]-GLP-1 (7-37); [Gly8, Glu30]-GLP-1 (7-37); [Val8, His37]-GLP-1 (7-37); [Gly8, His37]-GLP-1 (7-37); [Arg34]-GLP-1 (7-37); [Lys18]-GLP-1 (7-37); [Gly8, Glu22, Gly36]-GLP-1 (7-37); [Aib8, Aib22]-GLP-1 (7-37); [Aib8, Aib35]-GLP-1 (7-37); [Aib8, Aib22, Aib35]-GLP-1 (7-37); [Glu22, Glu23]-GLP-1 (7-37); [Gly8, Glu22, Glu23]-GLP-1 (7-37); [Val8, Glu22, Glu23]-GLP-1 (7-37); [Val8, Glu22, Val25]-GLP-1 (7-37); [Val8, Glu22, Ile33]-GLP-1 (7-37); [Val8, Glu22, Val25, Ile33]-GLP-1 (7-37); GLP-1(7-36) type thereof in which the position 37 is deleted; and GLP-1(7-35) type thereof in which the positions 36 and 37 are deleted.

(13) A method for improving biostability of a bioactive substance, comprising the step of binding the partial peptide according to any one of items (1) to (6) to a bioactive substance.
(14) The method according to the item (13), wherein the bioactive substance is GLP-1 or a derivative thereof, and the partial peptide is bound to a C-terminal of the GLP-1 or the derivative thereof.

**Advantageous Effects of Invention**

When the partial peptide of the GA module of the present invention is bound to a bioactive substance, the improvement of biostability such as stability in blood of the bioactive substance can be attained while the activity thereof is maintained. The partial peptide is particularly effective for gastrointestinal hormones and derivatives thereof as the bioactive substance.

**BRIEF DESCRIPTION OF DRAWINGS**

Fig.1 shows an amino acid sequence of a GA module, wherein GAm (1-46) shows a full length of the GA module 46 residues, GAm (17-46) shows a sequence from the position 17 to the position 46 of the GA module, and GAm (28-44) shows a sequence from the position 28 to the position 44 of the GA module.

Fig. 2 shows amounts of insulin secreted from MIN 6 cells with treated each GLP-1 peptide complex as relative values, supposing that an amount of insulin secreted from the cells with treated 1000 pM of native GLP-1 is 100%.

Fig. 3 shows amounts of insulin secreted from MIN 6 cells with treated each GLP-1 peptide complex as relative values, supposing that an amount of insulin secreted from the cells with treated 1000 pM of native GLP-1 is 100%.

Fig. 4 shows the results of blood glucose-lowering effect with time when each GLP-1 peptide complex is subcutaneously administered to db/db mice, which are type 2 diabetes mellitus model mice.

Fig. 5 shows the results of blood glucose-lowering effect with time when each GLP-1 peptide complex is subcutaneously administered to db/db mice, which are type 2 diabetes mellitus model mice.

## DESCRIPTION OF EMBODIMENTS

In the present description, the peptide depicted in the amino acid sequence has an N-terminal at the left end and a C-terminal at the right end in accordance with customary notation.

The partial peptide of the GA module refers to a peptide formed of a partial sequence of a GA module depicted in SEQ ID NO: 1. The "partial sequence of GA module" refers to a sequence formed of a part of the GA module sequence. Specifically, it refers to a fragment sequence in which single amino acid residue or continuous amino acid residues are deleted from one or both ends of the GA module depicted in SEQ ID NO: 1. The partial peptide of the GA module of the present invention is a peptide including at least five continuous amino acids defined by SEQ ID NO: 2, and having 5 to 25, more preferably 5 to 17 amino acids thereof. The partial peptide of the GA module of the present invention is depicted as the amino acid sequence as follows: formula:

$$Y_{22}\text{-}Y_{23}\text{-}Y_{24}\text{-}Y_{25}\text{-}Y_{26}\text{-}Y_{27}\text{-}Y_{28}\text{-}Y_{29}\text{-}Y_{30}\text{-}Y_{31}\text{-}Y_{32}\text{-}Y_{33}\text{-}Y_{34}\text{-}Y_{35}\text{-}Y_{36}\text{-}Y_{37}\text{-}Ile\text{-}Asp\text{-}Glu\text{-}Ile\text{-}Leu\text{-}Y_{43}\text{-}$$

$$Y_{44}\text{-}Y_{45}\text{-}Y_{46}$$

wherein: $Y_{22}$ is Lys or deletion; $Y_{23}$ is Asn or deletion; $Y_{24}$ is Leu or deletion; $Y_{25}$ is Ile or deletion; $Y_{26}$ is Asn or deletion; $Y_{27}$ is Asn or deletion; $Y_{28}$ is Ala or deletion; $Y_{29}$ is Lys or deletion; $Y_{30}$ is Thr or deletion; $Y_{31}$ is Val or deletion; $Y_{32}$ is Glu or deletion; $Y_{33}$ is Gly or deletion; $Y_{34}$ is Val or deletion; $Y_{35}$ is Lys or deletion; $Y_{36}$ is Ala or deletion; $Y_{37}$ is Leu or deletion; $Y_{43}$ is Ala or deletion; $Y_{44}$ is Ala or deletion; $Y_{45}$ is Leu or deletion; and $Y_{46}$ is Pro or deletion, provided that the deletion is limited to a continuous deletion from $Y_{22}$ and/or $Y_{46}$ (including single deletion of $Y_{22}$ or $Y_{46}$, and deletions at two portions of $Y_{22}$ and $Y_{46}$ alone).

It is known that the GA module has a high affinity to albumin, and it is sometimes called as ABD (Albumin binding domain). Sixteen kinds of peptide sequences are reported as the GA module in native bacteria (Biochemistry, 2006, 45 (10), 3263-3271). As one of the GA modules, there is a sequence which is a specific region in protein G of Streptococcus G148 strain (streptococcal protein G) and is composed of amino acid 46 resides, which is depicted in SEQ ID NO: 1. In Examples of the present invention, GA modules derived from streptococcus G 148 were used.

The partial peptide of the GA module of the present invention is a peptide which reinforces functions of a bioactive substance by being added to the bioactive substance. The substitution or addition site of the peptide depends on the bioactive substance, and an optimal site can be arbitrarily selected according to a known method on the basis of the properties and features of the bioactive substance. For example, for GLP-1, it is preferable to add the peptide to the C-terminal thereof, because the activity is remarkably impaired when the peptide is added to the N-terminal thereof. For amylin, the modification at N-terminal of the amylin is possible, because it is known that the activity is exhibited even if the N-terminal is modified (WO 2007/022123). In addition, for VIP, it is known that the activity is exhibited when the N-terminal is acetylated or converted into a cyclic peptide, or the C-terminal is modified with an amino acid, and the modification at C-terminal of the VIP is possible (WO2008/003612).

The term "bioactive substance" originally refers to substances controlling actions in vivo, but usually refers to a peptide, a proteins or a substance having very similar functions thereto, which can be utilized for the purpose of treatment or diagnosis of diseases. In usual, it may sometimes be called as a bioactive peptide, because there are many peptidic substances. The bioactive substance may include gastrointestinal hormones, growth hormones and derivatives thereof. The gastrointestinal hormone may include glucagon, GLP-1 (glucagon like peptide-1), GLP-2, GIP (gastric inhibitory polypeptide), gastrin, selectin, cholecystokinin, motilin, neurotensin, somatostatin, amylin, ghrelin, VIP (Vasoactive Intestinal Polypeptide), and the like. Here, the bioactive substance includes not only native substances but also synthetic products. In other words, it includes substances in which an amino acid which is a part of a native bioactive substance, is substituted or deleted, or to which an amino acid is added (for example inserted). Such a peptide design is performed for the purpose of enhancement of effects, enlargement of selectivity, and obtaining stability to peptide decomposition, and the like, and it can be carried out in a well-known method by those skilled in the art, though it depends on the bioactive substance. In addition, bioactive substances to which a sugar chain, fatty acid, lipid, nucleic acid, or the like is bound to a peptide chain may also be used.

Exendin-4 is a bioactive peptide which is found in a secretion in salivary gland of Heloderma Suspectum, and has GLP-1 like activity. In addition to the Exendin-4 (1-39), derivatives such as Exendin-4 (1-39)-LysLysLysLysLys, Exendin-4 (1-39)-LysLysLysLysLysLys are known, and they can be considered as a derivative of GLP-1.

The term "GLP-1 derivative" refers to a derivative in which a part of amino acids of GLP-1 (which may be sometimes called as a native GLP-1, in order to emphasize a difference from the GLP-1 derivative) are substituted or deleted, or to which the amino acids are added, and has substantially the same activity as that of GLP-1. In other words, the GLP-1 derivatives have preferably 50% to 150% activity of that of the native GLP-1. It is known that an enzyme resistance of the GLP-1 derivative is increased by the substitution at the position 8, at the positions 22 and 23, or at the positions 26 and 34. Besides this, the GLP-1 and GLP-1 derivatives are also described in WO91/11457, WO96/29342, WO98/08871, WO99/43341, WO99/43708, WO99/43707, WO99/43706, WO99/43705, WO00/07617, WO08/005527, WO00/34331, WO02/046227, WO02/098348, WO03/087139, WO03/018516, WO05/000892, WO05/027978, WO06/087354, and the like. It can be said from these documents that the GLP-1 derivatives having an amino acid sequence shown below are GLP-1 derivatives having substantially the same activity as that of the native GLP-1. As the native GLP-1 having the same activity, not only GLP-1 (7-36) but also GLP-1 (7-37) and GLP-1 (7-35) are known, and they have the same activity, regardless whether their C-terminals are an amide form or a carboxylic acid form.

The amino acid sequences of the GLP-1 derivatives is known as that depicting the following diversities.

$$X_7\text{-}X_8\text{-}X_9\text{-Gly-Thr-Phe-Thr-Ser-Asp-}X_{16}\text{-Ser-}X_{18}\text{-}X_{19}\text{-}X_{20}\text{-Glu-}X_{22}\text{-}X_{23}\text{-Ala-}X_{25}\text{-}X_{26}\text{-}X_{27}\text{-Phe-}$$

$$\text{Ile-}X_{30}\text{-Trp-Leu-}X_{33}\text{-}X_{34}\text{-}X_{35}\text{-}X_{36}\text{-}X_{37}\text{-}X_{38}\text{-}X_{39}\text{-}X_{40}\text{-}X_{41}\text{-}X_{42}\text{-}X_{43}\text{-}X_{44}\text{-}X_{45}$$

wherein: $X_7$ is His; $X_8$ is Ala, Gly, Ser, Thr, Leu, Ile, Val, Glu, Asp, Lys or Aib; $X_9$ is Glu, Gly, Asp or Lys; $X_{16}$ is Val, Ala, Gly, Ser, Thr, Leu, Ile, Tyr, Glu, Asp, Trp or Lys; $X_{18}$ is Ser, Ala, Arg, Gly, Thr, Leu, Ile, Val, Glu, Asp, Trp or Lys; $X_{19}$ is Tyr, Phe, Trp, Glu, Gln, Asp or Lys; $X_{20}$ is Leu, Met, Ala, Gly, Ser, Thr, Leu, Ile, Val, Glu, Asp, Trp, Tyr or Lys; $X_{22}$ is Gly, Ala, Glu, Ser, Thr, Leu, Ile, Val, Asp, Lys, Arg, Cys or Aib; $X_{23}$ is Gln, Arg, Glu, Asp, Asn or Lys; $X_{25}$ is Ala, Gly, Ser, Thr, Leu, Ile, Val, Glu, Asp or Lys; $X_{26}$ is Lys, Gln, Glu, Asp, His or Arg; $X_{27}$ is Glu, Ala, Asp, Lys or Leu; $X_{30}$ is Ala, Glu, Gly, Ser, Thr, Leu, Ile, Val, Asp, Lys, Gln, Tyr, His or Arg; $X_{33}$ is Val, Gly, Ala, Ser, Thr, Leu, Ile, Glu, Asp or Lys; $X_{34}$ is Lys, Arg, Glu, Asp, His, Ala, Gly or Asn; $X_{35}$ is Gly, Ala, Ser, Thr, Leu, Ile, Val, Glu, Asp, Lys, His, Pro or Aib; $X_{36}$ is Arg, Gly, Lys, Glu, Asp, His or deletion; $X_{37}$ is Gly, Pro, Glu, Lys, Ala, Thr, Ser, Asp, Leu, Ile, Val, His or deletion; $X_{38}$ is Ser, Lys, Arg, Glu, Asp, His or deletion; $X_{39}$ is Ser, Arg, Lys, Glu, Asp, His or deletion; $X_{40}$ is Gly, Glu, Lys, Asp or deletion; $X_{41}$ is Ala, Lys, Phe, Trp, Tyr, Glu, Asp or deletion; $X_{42}$ is Pro, Lys, Glu, Asp or deletion; $X_{43}$ is Pro, Lys, Glu, Asp or deletion; $X_{44}$ is Pro, Lys, Glu, Asp or deletion; and $X_{45}$ is Ser, Lys, Val, Glu, Asp or deletion.

Of these, GLP-1 having a sequence of $X_7$ to $X_{37}$ or the derivatives thereof ($X_7$ to $X_{37}$ are as described above) are preferable GLP-1 and derivatives thereof to which the partial peptide of the GA module of the present invention is applied.

As GLP-1 and the derivatives thereof, specifically the following substances are well known:

GLP-1 (7-37); [Ser8]-GLP-1 (7-37); [Gly8]-GLP-1 (7-37); [Val8]-GLP-1 (7-37); [Glu22]-GLP-1 (7-37); [Lys22]-GLP-1 (7-37); [Val8, Glu22]-GLP-1 (7-37); [Val8, Lys22]-GLP-1 (7-37); [Gly8, Glu22]-GLP-1 (7-37); [Gly8, Lys22]-GLP-1 (7-37); [Val8, Glu30]-GLP-1 (7-37); [Gly8, Glu30]-GLP-1 (7-37); [Val8, His37]-GLP-1 (7-37); [Gly8, His37]-GLP-1 (7-37); [Arg34]-GLP-1 (7-37); [Lys18]-GLP-1 (7-37); [Gly8, Glu22, Gly36]-GLP-1 (7-37); [Aib8, Aib22]-GLP-1 (7-37); [Aib8, Aib35]-GLP-1 (7-37); [Aib8, Aib22, Aib35]-GLP-1 (7-37); [Glu22, Glu23]-GLP-1 (7-37); [Gly8, Glu22, Glu23]-GLP-1 (7-37); [Val8, Glu22, Glu23]-GLP-1 (7-37); [Val8, Glu22, Val25]-GLP-1 (7-37); [Val8, Glu22, Ile33]-GLP-1 (7-37); [Val8, Glu22, Val25, Ile33]-GLP-1 (7-37); GLP-1(7-36) type thereof in which the position 37 is deleted; and GLP-1(7-35) type thereof in which the positions 36 and 37 are deleted.

The native VIP is known as a peptide of 28 amino acid residues, having the following sequence.

$$\text{His-Ser-Asp-Ala-Val-Phe-Thr-Asp-Asn-Tyr-Thr-Arg-Leu-Arg-Lys-Gln-Met-Ala-Val-Lys-Ly}$$

$$\text{s-Tyr-Leu-Asn-Ser-Ile-Leu-Asn-NH}_2$$

As the derivative of the VIP, PACAP-27 composed of His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-NH$_2$; PACAP-38 composed of His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-Gly-Lys-Arg-Tyr-Lys-Gln-Arg-Val-

Lys-Asn-Lys-NH$_2$; and RO25-1553 composed of N$^\alpha$-acetyl-His-Ser-Asp-Ala-Val-Phe-Thr-Glu-Asn-Tyr-Thr-Lys-Leu-Arg-Lys-Gln-Nle-Ala-A la-Lys-Lys*-Tyr-Leu-Asn-Asp*-Leu-Lys-Lys-Gly-Gly-Thr-NH$_2$ (in which Met at the position 17 is substituted to norleucine, and *: a lactam ring is formed between Lys at the position 21 and Asp at the position 25) are known.

The native somatostatin is known as a peptide of 14 amino acid residues, having the following sequence:

Ala-Gly-Cys-Lys-Asn-Phe-Phe-Trp-Lys-Thr-Phe-Thr-Ser-Cys-NH$_2$

Here, a disulfide bond is formed between the cysteine residues at the positions 3 and 14. It is possible to substitute Trp at the position 8 by D-Trp. In addition, a structural and functional analysis demonstrates that a β-turn fragment of Phe-Trp-Lys-Thr at the positions 7 to 10 of somatostatin is necessary for the activity [Nature, 1979, 280 (5722), 512-514; and Nature, 1981, 292 (5818), 55-58], and therefore it would appear that the amino acids at the positions 1 to 6 and the positions 11 to 14 can be deleted. Such a somatostatin derivative can be said to be a partial peptide of the native somatostatin including an amino acid sequence: Phe-Trp-Lys-Thr (continuous deletion from the N terminal and/or the C-terminal is possible). As other somatostatin derivatives, octreotide composed of D-Phe-Cys-Phe-D-Trp-Lys-Thr-Cys-Thr-ol ( a disulfide bond is formed between the cysteine residues), D-Phe-Phe-Phe-D-Trp-Lys-Thr-Phe-Thr, D-Phe-Cys-Tyr-D-Trp-Lys-Val-Cys-Thr (a disulfide bond is formed between the cysteine residues), and D-Phe-Cys-Tyr-D-Trp-Lys-Val-Cys-Thp (a disulfide bond is formed between the cysteine residues) are reported.

The native amylin is known as a peptide of 37 amino acid residues, having the following sequence:

Lys-Cys-Asn-Thr-Ala-Thr-Cys-Ala-Thr-Gln-Arg-Leu-Ala-Asn-Phe-Leu-Val-His-Ser-Ser-As

n-Asn-Phe-Gly-Ala-Ile-Leu-Ser-Ser-Thr-Asn-Val-Gly-Ser-Asn-Thr-Tyr-NH$_2$

Here, a disulfide bond is formed between the cysteine residues at the positions 2 and 7. It is possible to substitute Ala at the position 25 by Pro, Ser at the position 28 by Pro, and Ser at the position 29 by Pro. The substitution may be performed at single site or at optional multiple sites. A peptide analog in which GLP-1 is bound to the N-terminal is also known.

The native ghrelin is known as a peptide of 28 amino acid residues, having the following sequence:

Gly-Ser-Ser-Phe-Leu-Ser-Pro-Glu-His-Gln-Arg-Val-Gln-Gln-Arg-Lys-Glu-Ser-Lys-Lys-Pro-

Pro-Ala-Lys-Leu-Gln-Pro-Arg-NH$_2$

Here, it is known that the sufficient activity is secured by an amino acid sequence: Gly-Ser-Ser-Phe-Leu at the positions 1 to 5 of the ghrelin (Biochem Biophys Res Commun, 2001, 284(3), 655-659), and therefore it would appear that the amino acids at the positions 6 to 28 can be deleted. Such a ghrelin derivative can be said to be a partial peptide of the native ghrelin including an amino acid sequence: Gly-Ser-Ser-Phe-Leu (continuous deletion from the C-terminal is possible). In the ghrelin, Ser at the position 3 is octanoylated, but even if this octanoyl group is changed in the structure, it may be possible to maintain the activity (J Med Chem, 2000, 43 (23), 4370-4376).

In the present invention, the term "bioactive complex" refers to a substance in which a bioactive substance and the partial peptide of the GA module of the present invention are bound to each other. In usual, one molecule of the partial peptide of the GA module of the present invention is bound to one molecule of the bioactive substance. The C-terminal of the bioactive complex may be either an amide form or a coaroxylic acid form. This is because it is important for the bioactive substance to bind to the partial peptide of the GA module of the present invention to reinforce the biostability of the bioactive substance, regardless of the form of the C-terminal.

A short amino acid linker sequence may be inserted between the bioactive substance and the partial peptide of the GA module of the invention, as a method for binding the partial peptide of the GA module of the invention to the bioactive substance. The term "linker" refers to all things which can mainly serve as a spacer. The linker is preferably formed of amino acids linked with a peptide bond. Among them, the linkers formed of 1 to 3 amino acids are preferable, and Gly-Pro (GP) and Gly-Pro-Ser (GPS) are especially optimum.

In the present description, the term "biostability" refers to a stability of a substance in circulating blood, plasmas and serums collected, various living body tissues, and various tissue homogenates collected. As one index of the stabilities in blood, for example, an elimination half-life in blood (which may be sometimes called as a half-life in blood or half-life concentration in blood) is used, which is the time required to decrease the concentration of the substance to a half of the maximum concentration of the substance.

The method for synthesizing the partial peptide of the GA module or the bioactive complex of the present invention is not particularly limited, and conventional methods may be used. For example, chemical synthesis methods such as a solid phase synthesis and a liquid phase synthesis, synthetic methods using an enzyme, recombinant DNA technologies may be used. Any cell which can generate the peptide of the invention may be used as a host cell which introduces a DNA sequence or a recombinant vector, and the host cell may include bacteria, yeast, and eukaryotic cells which are more advanced. The host cells are exemplified by, for example, BHK or CHO cell lines. In all Examples described below, the solid synthesis methods with wide general-use are used, but the method is not necessarily limited thereto.

The GLP-1 peptide complex in which the partial peptide of the GA module of the present invention is applied to GLP-1 is applicable to various diseases against which GLP-1 receptor stimulation is effective. In other words, the GLP-1 peptide complex of the invention can be used for, for example, treatment of type 2 diabetes mellitus, treatment of type 1 diabetes mellitus, treatment of obesity and/or suppression of appetite, and the like. The administration amount of the GLP-1 peptide complex is appropriately decided by those skilled in the art according to an individual patient with a disease. In general, the administration amount can be thought to be from about 1 $\mu$g/day to about 10 mg/day. Exendin-4 may also be administered in an amount within the range described above. The GLP-1 peptide complex of the present invention has a high and prolonged stability *in vivo,* and therefore a dosage regimen can be determined based on the biostability of the individual GLP-1 peptide complex.

In addition, a bioactive complex in which the partial peptide of the GA module of the present invention is applied to VIP, somatostatin, amylin or ghrelin is applicable to various diseases against which each of the bioactive substances is effective. The administration amounts of these bioactive complexes are from about 50 $\mu$g/day to about 1 mg/day for VIP; from about 50 $\mu$g/day to about 1 mg/day for somatostatin; from about 5 $\mu$g/day to about 500 $\mu$g/day for amylin; and from about 100 $\mu$g/day to about 1 mg/day for ghrelin. When the partial peptide of the GA module of the present invention is applied to a bioactive substance other than the above, the administration amount of such a complex can easily be determined based on the known administration amount of the bioactive substance. In other words, the administration amount is set as the equivalent amount measured in moles, from which an optimal value may be selected.

The bioactive complex of the present invention can be administered through various administration routes. In general, it is administered subcutaneously, intravenously or pulmonarily to a patient who requires the treatment. In addition, an administration route is appropriately selected from an oral administration, nasal mucosal administration, epidermal administration, ophthalmic administration, and the like, depending on the application range, the nature of the bioactive complex and the combination of administration techniques. The already-known administration route of the bioactive substance may be selected.

**Examples**

The present invention will be explained in more detail by Experimental Examples described below, but the present invention is not limited thereto, and any modification may be made within the scope of the present invention.

I Synthesis of GLP-1 Peptide Complex

GLP-1 was selected as a bioactive substance having low biostability, and was inspected. The partial peptide of the GA module of the present invention was added to the C-terminal of GLP-1 or the GLP-1 derivative to form a GLP-1 peptide complex. The GLP-1 peptide complex was synthesized in a solid synthesis method, and a synthesized product was confirmed by a mass spectrometry after the purification using an HPLC. -$NH_2$ depicts that the C-terminal was amidated.

GLP-1 (7-35) was mainly used as GLP-1, the position 8 of which was a native alanine, or was substituted by serine or glycine. [Ser8] depicts that the alanine at the second position from the left of the native GLP-1 depicted by SEQ ID NO: 24, i.e., at the position 8, was changed by serine, and has the same meaning as 8S. In this field, the N-terminal of the left end is generally defined as the position 7 in the native GLP-1, because the N-terminal of the GLP-1 precursor is defined as the position 1.

A GA module of 46 residues depicted by SEQ ID NO: 1, or a partial peptide thereof was used as a peptide to be added. The peptide depicted by SEQ ID NO: 1 was abbreviated as GAm (1-46), because it was a peptide having a sequence starting with an amino acid at the first position of the GA module, and ending with an amino acid at the 46th position. The partial peptide of the GA module was abbreviated as GAm (X-Y), which was a peptide having a sequence starting with an amino acid at the position X of the GA module depicted by SEQ ID NO: 1, and ending with an amino acid at the position Y.

SEQ ID NO: 1 to SEQ ID NO: 23 depict the peptides composed of the complete sequence or partial sequence of the GA module, and SEQ ID NO: 24 to SEQ ID NO: 58 depict the GLP-1 or derivative thereof, which are bioactive substances.

Example 1. [Ser8]-GLP-1(7-35)-GAm(38-42)-$NH_2$ SEQ ID NO: 28 (GAm: SEQ ID NO: 2)
Example 2. [Ser8]-GLP-1(7-35)-GAm(38-44)-$NH_2$ SEQ ID NO: 29 (GAm: SEQ ID NO: 3)

Example 3. [Ser8]-GLP-1(7-35)-GAm(37-44)-NH$_2$ SEQ ID NO: 30 (GAm: SEQ ID NO: 4)
Example 4. [Ser8]-GLP-1(7-35)-GAm(35-44)-NH$_2$ SEQ ID NO: 31 (GAm: SEQ ID NO: 5)
Example 5. GLP-1(7-35)-GAm(38-42)-NH$_2$ SEQ ID NO: 32 (GAm: SEQ ID NO: 2)
Example 6. GLP-1(7-35)-GAm(35-44)-NH2 SEQ ID NO: 33 (GAm: SEQ ID NO: 5)
Example 7. GLP-1(7-35)-GAm(33-43)-NH$_2$ SEQ ID NO: 34 (GAm: SEQ ID NO: 7)
Example 8. GLP-1(7-35)-GAm(33-44)-NH$_2$ SEQ ID NO: 35 (GAm: SEQ ID NO: 8)
Example 9. [Ser8]-GLP-1(7-35)-GAm(33-44)-NH$_2$ SEQ ID NO: 36 (GAm: SEQ ID NO: 8)
Example 10. [Ser8]-GLP-1(7-35)-GAm(32-44)-NH2 SEQ ID NO: 37 (GAm: SEQ ID NO: 9)
Example 11. [Ser8]-GLP-1(7-35)-GAm(31-44)-NH$_2$ SEQ ID NO: 38 (GAm: SEQ ID NO: 10)
Example 12. [Ser8]-GLP-1(7-35)-GAm(30-42)-NH$_2$ SEQ ID NO: 39 (GAm: SEQ ID NO: 11)
Example 13. [Ser8]-GLP-1(7-35)-GAm(28-42)-NH$_2$ SEQ ID NO: 40 (GAm: SEQ ID NO: 12)
Example 14. [Ser8]-GLP-1(7-35)-GAm(28-44)-NH$_2$ SEQ ID NO: 41 (GAm: SEQ ID NO: 13)
Example 15. [Ser8]-GLP-1(7-35)-GAm(27-46)-NH$_2$ SEQ ID NO: 42 (GAm: SEQ ID NO: 14)
Example 16. [Ser8]-GLP-1(7-35)-GAm(25-46)-NH$_2$ SEQ ID NO: 43 (GAm: SEQ ID NO: 15)
Example 17. [Ser8]-GLP-1(7-35)-GAm(22-46)-NH$_2$ SEQ ID NO: 44 (GAm: SEQ ID NO: 16)
Example 18. [Ser8]-GLP-1(7-35)-GAm(28-44) SEQ ID NO: 41 (GAm: SEQ ID NO: 13)
Example 19. [Ser8]-GLP-1(7-35)-GAm(33-44) SEQ ID NO: 36 (GAm: SEQ ID NO: 8)
Example 20. [Ser8]-GLP-1(7-35)-GAm(37-44) SEQ ID NO: 30 (GAm: SEQ ID NO: 4)
Example 21. GLP-1(7-35)-Gly-Pro-GAm(35-44)-NH$_2$ SEQ ID NO: 45 (GAm: SEQ ID NO: 5)
Example 22. GLP-1(7-35)-Gly-Pro-GAm(34-44)-NH$_2$ SEQ ID NO: 46 (GAm: SEQ ID NO: 6)
Example 23. GLP-1(7-35)-Gly-Pro-Ser-GAm(34-44)-NH$_2$ SEQ ID NO: 47 (GAm: SEQ ID NO:6)
Example 24. GLP-1(7-35)-Gly-Pro-Ser-GAm(33-44)-NH$_2$ SEQ ID NO: 48 (GAm: SEQ ID NO: 8)
Comparative Example 1. GLP-1(7-36)-NH$_2$ SEQ ID NO: 24
Comparative Example 2. [Ser8]-GLP-1(7-36)-NH$_2$ SEQ ID NO: 26
Comparative Example 3. Exendin-4(1-39)-NH$_2$ SEQ ID NO: 27
Comparative Example 4. [Ser8]-GLP-1(7-35)-GAm(1-46)-NH$_2$ SEQ ID NO: 49(GAm:SEQ ID NO: 1)
Comparative Example 5. [Ser8]-GLP-1(7-35)-GAm(17-46)-NH$_2$ SEQ ID NO: 50(GAm:SEQ ID NO: 17)
Comparative Example 6. [Ser8]-GLP-1(7-35)-GAm(21-46)-NH$_2$ SEQ ID NO: 51(GAm:SEQ ID NO: 18)
Comparative Example 7. [Ser8]-GLP-1(7-35)-GAm(28-40)-NH$_2$ SEQ ID NO: 52(GAm:SEQ ID NO: 19)
Comparative Example 8. [Ser8]-GLP-1(7-35)-GAm(28-37)-NH$_2$ SEQ ID NO: 53(GAm:SEQ ID NO: 20)
Comparative Example 9. [Ser8]-GLP-1(7-35)-GAm(39-44)-NH$_2$ SEQ ID NO: 54(GAm:SEQ ID NO: 21)
Comparative Example 10. GLP-1(7-35)-GAm(38-41)-NH$_2$ SEQ ID NO: 55(GAm:SEQ ID NO: 22)
Comparative Example 11. GLP-1(7-35)-GAm(38-40)-NH$_2$ SEQ ID NO: 56(GAm:SEQ ID NO: 23)
Comparative Example 12. [Gly8]-GLP-1(7-35)-RPSS-NH$_2$ SEQ ID NO: 57
Comparative Example 13. [Ser8,Glu22,Glu23]-GLP-1(7-35)-GPSSGAPPPS-NH$_2$ SEQ ID NO: 58

<Experimental Example 1> Evaluation of Plasma Stability of GLP-1 Peptide Complex

The stability in plasma was evaluated *in vitro,* in order to inspect the biostability of the GLP-1 peptide complex.

1. Procedure

SD rats were anesthetized with diethyl ether, whole blood was collected and a plasma fraction was separated therefrom. Each GLP-1 peptide complex was added to the plasma so that the final concentration was 0.5 μmol/L, and the mixture was incubated at 37°C. A sample was recovered after 0, 8 or 48 hours, and a remaining rate was evaluated by HPLC/MS measurement of an amount of the GLP-1 peptide complex. The remaining rate of the GLP-1 peptide complex was evaluated by calculating a rate of an HPLC/MS measurement result of the complex remaining in the plasma recovered after 8 hours or 48 hours relative to an HPLC/MS measurement result of the complex in the sample recovered at 0 hour, which was assumed as 100%.

2. Results and Discussion

The analysis results of the plasma stability of the GLP-1 peptide complexes are shown in Table 1 and Table 2 as a remaining rate.

(1) Effect of Partial Peptide of GA Module

The native GLP-1 in which the peptide was not added (Comparative Example 1), and 8S-GLP-1 (Comparative Example 2) were completely decomposed in the plasma after 48 hours. The remaining rates of the GLP-1 peptide complexes in which the peptide which was not the GA module sequence was added (Comparative Examples 12 and 13) were 0% and 12% in the plasma after 48 hours. They had a remarkably low plasma stability which was nearly equal to the native GLP-1 (Comparative Example 1) or the 8S-GLP-1 (Comparative Example 2).

On the other hand, the GLP-1 peptide complexes in which the partial peptide of the GA module was added (Example 1 to Example 20) remained in a state where they were hardly decomposed in the rat plasma after 48 hours, and they obtained a remarkably higher plasma stability than those of the native GLP-1 (Comparative Example 1) and the 8S-GLP-1 (Comparative Example 2). As shown in Examples 5 to 8, it became apparent that the plasma stability equal to that obtained in the case of the addition to the 8S-GLP-1 could be obtained, even if the peptide described above was added to the native GLP-1. It is surprising to exhibit such results even though the native GLP-1 has the remarkably low plasma stability. In Examples 18 to 20, the carboxylic acid forms at the C-terminal were used, but they could obtain the equal effect to that obtained in the amide form.

With respect to the chain length of the GA module, the GLP-1 peptide complexes in which a long-chain GAm (1-46) and GAm (17-46) were added respectively (Comparative Examples 4 and 5) exhibited a remarkably high plasma stability, i.e., a remaining rate of 105% in the plasma after 48 hours. In addition, GLP-1 peptide complexes in which the GAm (38-42) was added (Examples 1 and 5) exhibited, surprisingly, remarkably high plasma stabilities, i.e., remaining rates of 71% and 83%, respectively, in the plasma after 48 hours. It became apparent that even the partial peptide of the GA module of five residues could impart the plasma stability nearly equal to that obtained from the partial peptide of the GA module of 30 residues or the full length peptide of the GA module.

On the contrary, the GLP-1 peptide complexes in which GAm (39-44), GAm (38-41) and GAm (38-40) were added respectively (Comparative Examples 9, 10 and 11) were completely decomposed, i.e., all of the remaining rates were 0% in the plasma after 48 hours. The GLP-1 peptide complexes in which the GAm (28-40) and the GAm (28-37) were added respectively (Comparative Examples 7 and 8) were almost completely decomposed, i.e., the remaining rates were 4% and 0% respectively, in the plasma after 48 hours. This demonstrates that Ile at the position 38 and Leu at the position 42 are essential for obtaining the plasma stability in the partial peptide of the GA module. In other words, it was shown that the minimum amino acid sequence necessary for obtaining the plasma stability was the five residues: Ile-Asp-Glu-Ile-Leu (IDEIL) depicted by the GAm (38-42).

(2) Addition of Partial Peptide of GA Module of the Invention through Linker

In Example 21 to Example 24, the GLP-1 peptide complexes in which the partial peptide of the GA module was added through a linker were shown. These GLP-1 peptide complexes also exhibited sufficient remaining rates in the plasma after 48 hours. This demonstrated that the plasma stability could be secured even if the partial peptide of the GA module was added through the linker.

[Table 1]

| Plasma Stability | | | |
|---|---|---|---|
| Number (GLP-1 peptide complex abbreviated name) | Partial peptide sequence of GA module (additional sequence) | *Remaining rate* in plasma after 8 hours (%) | *Remaining rate* in plasma after 48 hours (%) |
| Example 1 8S-GLP-1 (7-35)-GAm (38-42)-NH$_2$ | IDEIL | 96 | 71 |
| Example 2 8S-GLP-1 (7-35)-GAm (38-44)-NH$_2$ | IDEILAA | 97 | 95 |
| Example 3 8S-GLP-1 (7-35)-GAm (37-44)-NH$_2$ | LIDEILAA | 113 | 95 |

(continued)

| Plasma Stability | | | |
|---|---|---|---|
| Number (GLP-1 peptide complex abbreviated name) | Partial peptide sequence of GA module (additional sequence) | *Remaining rate* in plasma after 8 hours (%) | *Remaining rate* in plasma after 48 hours (%) |
| Example 4 8S-GLP-1 (7-35)-GAm (35-44)-NH$_2$ | KALIDEILAA | 77 | 117 |
| Example 5 GLP-1 (7-35)-GAm (38-42)-NH$_2$ | IDEIL | 73 | 83 |
| Example 6 GLP-1 (7-35)-GAm (35-44)-NH$_2$ | KALIDEILAA | 97 | 75 |
| Example 7 GLP-1 (7-35)-GAm (33-43)-NH$_2$ | GVKALIDEILA | 94 | 87 |
| Example 8 GLP-1 (7-35)-GAm (33-44)-NH$_2$ | GVKALIDEILAA | 102 | 76 |
| Example 9 8S-GLP-1 (7-35)-GAm (33-44)-NH$_2$ | GVKALIDEILAA | 104 | 93 |
| Example 10 8S-GLP-1 (7-35)-GAm (32-44)-NH$_2$ | EGVKALIDEILAA | 96 | 68 |
| Example 11 8S-GLP-1 (7-35)-GAm (31-44)-NH$_2$ | VEGVKALIDEILAA | 99 | 92 |
| Example 12 8S-GLP-1 (7-35)-GAm (30-42)-NH$_2$ | TVEGVKALIDEIL | 105 | 116 |
| Example 13 8S-GLP-1 (7-35)-GAm (28-42)-NH$_2$ | AKTVEGVKALIDEIL | 88 | 87 |
| Example 14 8S-GLP-1 (7-35)-GAm (28-44)-NH$_2$ | AKTVEGVKALIDEILAA | 107 | 116 |
| Example 15 8S-GLP-1 (7-35)GAm (27-46)-NH$_2$ | NAKTVEGVKALIDEILAALP | 111 | 93 |
| Example 16 8S-GLP-1 (7-35)-GAm (25-46)-NH$_2$ | INNAKTVEGVKALIDEILAALP | 102 | 93 |
| Example 17 8S-GLP-1 (7-35)-GAm (22-46)-NH$_2$ | KNLINNAKTVEGVKALIDEILAALP | 80 | 93 |
| Example 18 8S-GLP-1 (7-35)-GAm(28-44) | AKTVEGVKALIDEILAA | 99 | 88 |

(continued)

| Plasma Stability | | | |
|---|---|---|---|
| Number (GLP-1 peptide complex abbreviated name) | Partial peptide sequence of GA module (additional sequence) | *Remaining rate* in plasma after 8 hours (%) | *Remaining rate* in plasma after 48 hours (%) |
| Example 19 8S-GLP-1 (7-35)-GAm(33-44) | GVKALIDEILAA | 114 | 102 |
| Example 20 8S-GLP-1 (7-35)-GAm(37-44) | LIDEILAA | 125 | 79 |
| Example 21 GLP-1 (7-35)-GP-GAm (35-44)-NH$_2$ | (GP)+KALIDEILAA | 86 | 53 |
| Example 22 GLP-1 (7-35)-GP-GAm (34-44)-NH$_2$ | (GP)+VKALIDEILAA | 98 | 94 |
| Example 23 GLP-1 (7-35)-GPS-GAm (34-44)-NH$_2$ | (GPS)+VKALIDEILAA | 92 | 57 |
| Example 24 GLP-1 (7-35)-GPS-GAm (33-44)-NH$_2$ | (GPS)+GVKALIDEILAA | 90 | 40 |

[Table 2]

| Plasma Stability | | | |
|---|---|---|---|
| Number (GLP-1 peptide complex abbreviated name) | Partial peptide sequence of GA module (additional sequence) | Remaining rate in plasma after 8 hours (%) | Remaining rate in plasma after 48 hours (%) |
| Comparative Example 1 GLP-1(7-36)-NH$_2$ | | 0 | 0 |
| Comparative Example 2 8S-GLP-1(7-36)-NH$_2$ | | 17 | 0 |
| Comparative Example 3 Exendin-4(1-39)-NH$_2$ | | 20 | 0 |
| Comparative Example 4 8S-GLP-1(7-35)-GAm (1-46)-NH$_2$ | LAEAKVLANRELDKYG VSDYYKNLINNAKTVEGVKALIDEILAALP | | 105 |
| Comparative Example 5 8S-GLP-1(7-35)-GAm (17-46)-NH$_2$ | VSDYYKNLINNAKTVEGVKALIDEILAALP | 105 | 105 |
| Comparative Example 6 8S-GLP-1(7-35)-GAm (21-46)-NH$_2$ | YKNLINNAKTVEGVKALIDEILAALP | | 84 |
| Comparative Example 7 8S-GLP-1(7-35)-GAm (28-40)-NH$_2$ | AKTVEGVKALIDE | 81 | 4 |

(continued)

| Plasma Stability | | | |
|---|---|---|---|
| Number (GLP-1 peptide complex abbreviated name) | Partial peptide sequence of GA module (additional sequence) | Remaining rate in plasma after 8 hours (%) | Remaining rate in plasma after 48 hours (%) |
| Comparative Example 8 8S-GLP-1(7-35)-GAm (28-37)-NH$_2$ | AKTVEGVKAL | 27 | 0 |
| Comparative Example 9 8S-GLP-1(7-35)-GAm (39-44)-NH$_2$ | DEILAA | 63 | 0 |
| Comparative Example 10 GLP-1(7-35)-GAm (38-41)-NH$_2$ | IDEI | 35 | 0 |
| Comparative Example 11 GLP-1(7-35)-GAm (38-40)-NH$_2$ | IDE | 0 | 0 |
| Comparative Example 12 8G-GLP-1(7-35)-RPSS-NH$_2$ | (RPSS) | 34 | 0 |
| Comparative Example 13 8S,22,23E-GLP-1 (7-35)-GPSSGAPPPS-NH$_2$ | (OPSSGAPPPS) | 57 | 12 |

<Experimental Example 2> Comparison in Activity Intensity of GLP-1 Peptide Complex

An insulin secretion capacity was analyzed *in vitro,* in order to evaluate the activity intensity of the GLP-1 peptide complexes exhibiting the high plasma stability in Experimental Example 1.

1. Procedure

The insulin secretion capacity was evaluated using MIN 6 (obtained from Mr. Junichi Miyazaki) which was an insulinoma cell derived from a mouse, expressing an endogenous GLP-1 receptor. MIN 6 was seeded on a multi-plate, which was cultivated at 37°C for 48 hours. After that, the culture was washed twice with a KRH buffer solution including 2 mM glucose solution (including NaCl 119 mM, KCI 4.74 mM, CaCl$_2$ 2.54 mM, MgCl$_2$ 1.19 mM, KH$_2$PO$_4$ 1.19 mM, 10 mM HEPES buffer solution pH7.3, and 0.1 % BSA), and then it was incubated at 37°C for one hour. After aspiration and removal of the solution above, a 15 mM glucose-containing KRH buffer solution including the GLP-1 peptide complex was added thereto, and the resulting mixture was incubated at 37°C for one hour. A supernatant thereof was recovered, and an amount of insulin secreted was measured. The measurement of the insulin was performed in an enzyme-linked immunosorbent assay (ELISA), and an absorbance was measured at 450 nm (a sub-wavelength: 620 nm) after the addition of a substrate. The test was composed of the following four parts.

[Part 1]

The activities of the native GLP-1 (Comparative Example 1) and 8S-GLP-1-GAm (1-46) (Comparative Example 4) were evaluated in a concentration of 30, 100, 300 or 1000 pM, and EC$_{50}$ values were calculated.

[Part 2]

The EC$_{50}$ values of the native GLP-1 (Comparative Example 1), 8S-GLP-1-GAm (17-46) (Comparative Example 5) and 8S-GLP-1-GAm (28-44) (Example 14) were calculated in the same manner as in Part 1.

[Part3]

The activity of each GLP-1 peptide complex was evaluated in a concentration of 1000 pM, which concentration was thought to be necessary for exhibiting the maximum activity of the native GLP-1 in the MIN 6 cells, and the obtained activity was compared with the activity intensity of the native GLP-1.

[Part4]

8S-GLP-1-GAm (32-44) (Example 10: GAm 13 residues), GLP-1-GAm (33-44) (Example 8: GAm 12 residues), GLP-1-GAm (35-44) (Example 6: GAm 10 residues) and GLP-1-GPS-GAm (34-44) (Example 22: GAm 11 residues) were selected as a GLP-1 peptide complex in which a partial peptide of a medium-chain type GA module was added, and 8S-GLP-1-GAm (38-44) (Example 2: GAm 7 residues) and 8S-GLP-1-GAm (38-42) (Example 1: GAm 5 residues) were selected as a GLP-1 peptide complex in which a partial peptide of a short-chain type GA module was added. $EC_{50}$ values thereof were calculated in the same manner as in Part 2, and the influence on the bioactivity caused by the addition of the partial peptide of the GA module were inspected in detail.

2. Results and Discussion

[Part 1]

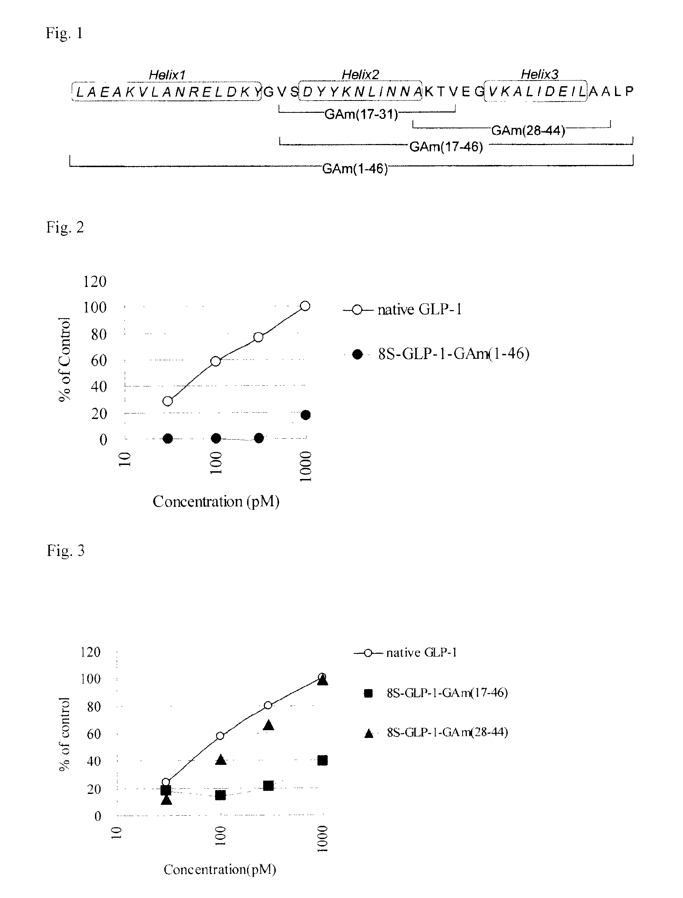

The insulin secretion capacities of the GLP-1 peptide complex: 8S-GLP-1-GAm (1-46) in which the full length GA module, GAm (1-46), was added (Comparative Example 4) and the native GLP-1 (Comparative Example 1) are shown in Fig. 2. As shown therein, it became apparent that the activity of the 8S-GLP-1-GAm (1-46) (Comparative Example 4) was remarkably impaired compared with the native GLP-1 (Comparative Example 1).

[Part 2]

Next, the insulin secretion capacities of the GLP-1 peptide complex in which the partial peptide of the GA module was added and the native GLP-1 (Comparative Example 1) are shown in Fig. 3. The activity of the 8S-GLP-1-Gam (17-46) (Comparative Example 5), the GLP-1 peptide complex in which the partial peptide of the GA module of 30 residues was added, was hardly detected, and even if the treatment at the maximum concentration of 1000 pM was performed, the complex could exhibit the activity of only less than 40% of the maximum activity of the native GLP-1 (Comparative Example 1). On the other hand, the GLP-1 peptide complex in which the partial peptide of the GA module of 17 residues was added, the 8S-GLP-1-GAm (28-44) (Example 14), maintained almost the same activity as that of the native GLP-1.
From the experiments performed above, it became apparent that when the partial peptide of the long-chain GA module having 30 or more residues was added, the plasma stability of the GLP-1 peptide complex was improved, but the bioactivity thereof was remarkably impaired.
A concentration ($EC_{50}$) of each GLP-1 peptide complex necessary for exhibiting 50% of the maximum activity of the native GLP-1 was analyzed based on the results shown in Fig. 2 and Fig. 3, and the values are shown in Table 3. The analysis was performed using the following calculation formula. In control, the insulin secretion capacity of the native GLP-1 in a concentration of 1000 pM was set as the maximum activity.

$$\text{Control} = (\text{an amount of insulin secreted from the native GLP-1 in a concentration of 1000 pM}) - (\text{an amount of insulin secreted in 15 mM glucose})$$

$$\text{Activity Intensity (\%)} = [\{(\text{an amount of insulin secreted from each GLP-1 peptide complex}) - (\text{an amount of insulin secreted in 15mM glucose})\}/\text{control}] \times 100$$

$EC_{50}$ value: A concentration of the GLP-1 peptide complex necessary for exhibiting 50% of Control
It is also apparent from the $EC_{50}$ values that the activities of the 8S-GLP-1-GAm (1-46) (Comparative Example 4) and the 8S-GLP-1-GAm (17-46) (Comparative Example 5), among the GLP-1 peptide complexes in which the full length GA module or the partial peptide thereof was added, were remarkably decreased compared with that of the 8S-GLP-1-GAm (28-44) (Example 14).
[Table 3]

| Insulin Secretion Capacity of GLP-1 Peptide Complex in MIN 6 Cells (EC$_{50}$ value) | | |
|---|---|---|
| Number (GLP-1 peptide complex abbreviated name) | Partial peptide sequence of GA module | EC$_{50}$ value (nM) |
| Comparative Example 1 GLP-1(7-36)-NH$_2$ | | 0.12 |
| Comparative Example 4 8S-GLP-1(7-35)-GAm (1-46)-NH$_2$ | LAEAKVLANRELDKYGVSDYYKNLINNAKTVEGVKALIDEILAALP | >1 |
| Comparative Example 5 8S-GLP-1(7-35)-GAm (17-46)-NH$_2$ | VSDYYKNLINNAKTVEGVKALIDEILAALP | >1 |
| Example 14 8S-GLP-1 (7-35)-GAm(28-44)-NH$_2$ | AKTVEGVKALIDEILAA | 0.21 |

[Part 3]

The results of comparison of the activity intensity between each GLP-1 peptide complex and the native GLP-1 (Example 1) in a concentration of 1000 pM are shown in Table 4 and Table 5. The 8S-GLP-1-GAm (22-46) in which the partial peptide of the GA module of 25 residues was added (Example 17) had activity equal to that of the native GLP-1 (Comparative Example 1), whereas the activity intensity of the 8S-GLP-1-GAm (21-46) in which the partial peptide of the GA module of 26 residues was added (Comparative Example 6) had a remarkably decreased activity of 45%. It was shown that the GLP-1 peptide complex in which the partial peptide of the GA module of 25 or less residues was added (Example 1 to Example 24) had the activity equal to that of the native GLP-1 (Comparative Example 1).

[Table 4]

| Insulin Secretion Capacity of GLP-1 Peptide Complex in MIN 6 Cells (% of control) | | |
|---|---|---|
| Number (GLP-1 peptide complex abbreviated name) | Partial peptide sequence ofGA module (additional sequence) | % of control |
| Example 1 8S-GLP-1(7-35)-GAm(38-42)-NH$_2$ | IDEIL | 125 |
| Example 2 SS-GLP-1(7-35)-GAm(38-44)-NH$_2$ | IDEILAA | 120 |
| Example 3 8S-GLP-1(7-35)-GAm(37-44)-NH$_2$ | LIDEILAA | 82 |
| Example 4 8S-GLP-1(7-35)-GAm(35-44)-NH$_2$ | KALIDEILAA | 115 |
| Example 5 GLP-1(7-35)-GAm(38-42)-NH$_2$ | IDEIL | 112 |
| Example 6 GLP-1(7-35)-GAm(35-44)-NH$_2$ | KALIDEILAA | 106 |
| Example 7 GLP-1(7-35)-GAm(33-43)-NH$_2$ | GVKALIDEILA | 105 |
| Example 8 GLP-1(7-35)-GAm(33-44)-NH$_2$ | GVKALIDEILAA | 99 |
| Example 9 8S-GLP-1(7-35)-GAm(33-44)-NH$_2$ | GVKALIDEILAA | 118 |
| Example 10 8S-GLP- 1(7-35)-GAm(32-44)-NH$_2$ | EGVKALIDEILAA | 109 |
| Example 11 8S-GLP-1(7-35)-GAm(31-44)-NH$_2$ | VEGVKALIDEILAA | 83 |
| Example 12 8S-GLP-1(7-35)-GAm(30-42)-NH$_2$ | TVEGVKALIDEIL | 85 |
| Example 13 8S-GLP-1(7-35)-GAm(28-42)-NH$_2$ | AKTVEGVKALIDEIL | 105 |
| Example 14 8S-GLP-1(7-35)-GAm(28-44)-NH$_2$ | ARTVEGVKALIDEILAA | 105 |
| Example 15 8S-GLP-1(7-35)-GAm(27-46)-NH$_2$ | NAKTVEGVKALIDEILAALP | 106 |

(continued)

| Insulin Secretion Capacity of GLP-1 Peptide Complex in MIN 6 Cells (% of control) | | |
|---|---|---|
| Number (GLP-1 peptide complex abbreviated name) | Partial peptide sequence ofGA module (additional sequence) | % of control |
| Example 16 8S-GLP-1(7-35)-GAm(25-46)-NH$_2$ | INNAKTVEGVKALIDEILAALP | 104 |
| Example 17 8S-GLP-1(7-35)-GAm(22-46)-NH$_2$ | KNLINNAKTVEGVKALIDEILAALP | 100 |
| Example 18 8S-GLP-1(7-35)-GAm(28-44) | AKTVEGVKALIDEILAA | 139 |
| Example 19 8S-GLP-1(7-35)-GAm(33-44) | GVKALIDEILAA | 89 |
| Example 20 8S-GLP-1(7-35)-GAm(37-44) | LIDEILAA | 98 |
| Example 21 GLP-1(7-35)-GP-GAm(35-44)-NH$_2$ | (GP)+KAUDEILAA | 103 |
| Example 22 GLP-1(7-35)-GP-GAm(34-44)-NH$_3$ | (GP)+VKALIDEILAA | 99 |
| Example 23 GLP-1(7-35)-GPS-GAm (34-44)-NH$_2$ | (GPS)+VKALIDEILAA | 103 |
| Example 24 GLP-1(7-35)-GPS-GAm (33-44)-NH$_2$ | (GPS)+GVKALIDEILAA | 123 |

[Table 5]

| Insulin Secretion Capacity of GLP-1 Peptide Complex in MIN 6 Cells (% of control) | | |
|---|---|---|
| Number (GLP-1 peptide complex abbreviated name) | Partial peptide sequence of GA module | % of control |
| Comparative Example 1 GLP-1 (7-36)-NH$_2$ | | 100 |
| Comparative Example 2 85-GLP-1(7-36)-NH$_2$ | | 83 |
| Comparative Example 4 8S-GLP-1(7-35)-GAm (1-46)-NH$_2$ | LAEAKVLANRELDKYGVSDYYKNLINNAKTVEGVKALIDEILAALP | 18 |
| Comparative Example 5 8S-GLP-1(7-35)-GAM (17-46)-NH$_2$ | VSDYYKNLINNAKTVEGVKALIDEILAALP | 39 |
| Comparative Example 6 8S-GLP-1(7-35)-GAm (21-46)-NH$_2$ | YKNLINNAKTVEGVKALIDEILAALP | 45 |

[Part4]

As shown in Table 6, it was shown from the EC$_{50}$ values that the GLP-1 peptide complexes in which the partial peptide of either the medium-chain type or the short-chain type GA module of the present invention was added had sufficient activity which compares favorably with the native GLP-1. The results above show that when the partial peptide of the GA module of the present invention is added to the bioactive substance, the high biostability can be obtained while the bioactivity is maintained.

[Table 6]

| Insulin Secretion Capacity of GLP-1 Peptide Complex in MIN 6 Cells (EC$_{50}$ value) | | |
|---|---|---|
| Number (GLP-1 peptide complex abbreviated name) | Partial peptide sequence ofGA module (additional sequence) | EC$_{50}$ value (nM) |
| Example 1 8S-GLP-1(7-35)-GAm(38-42)-NH$_2$ | IDEIL | 0.50 |
| Example 2 8S-GLP-1(735)-GAm(38-44)-NH$_2$ | IDEILAA | 0.27 |
| Example 6 GLP-1(7-35)-GAm(35-44)-NH$_2$ | KALIDEILAA | 0.09 |
| Example 8 GLP-1(7-35)-GAm(33-44)-NH$_2$ | GVKALIDEILAA | 0.55 |
| Example 10 8S-GLP-1(7-35)-GAm (32-44)-NH$_2$ | EGVKALIDEILAA | 0.12 |
| Example 23 GLP-1(7-35)-GPS-GAm (34-44)-NH$_2$ | (GPS)+VKALIDEILAA | 0.34 |
| Comparative Example 1 GLP-1(7-36)-NH$_2$ | | 0.12 |
| Comparative Example 2 8S-GLP-1(7-36)-NH$_2$ | | 0.18 |

<Experimental Example 3> Sustained Blood Glucose-Lowering Effect of GLP-1 Peptide Complex in Type 2 Diabetes Mellitus Model Mice

1. Procedure

The improvement of the biostability was evaluated using a duration time of blood glucose-lowering effect *in vivo* as an indicator. That is to say, db/db mice which were type 2 diabetes mellitus model mice (Charles River Laboratories Japan, Inc.) were separately bred, and each GLP-1 peptide complex was subcutaneously administered there to, and then a sustained change in a blood glucose level was inspected. The GLP-1 peptide complex was prepared to a con-centration of 100 $\mu$M, and was stored at -80°C until the test was performed. The GLP-1 peptide complex was diluted with saline to a pre-determined concentration and used in the test. The volume of a drug solution was 10 ml/kg, and either GLP-1 peptide complex was subcutaneously administered in a concentration of 50 nmol/kg. In order to measure the blood glucose level, the blood was sampled from a tail using a glass tube treated with heparin before and at a pre-determined time after the administration of the GLP-1 peptide complex (30 minutes, 1 hour, 2 hours, 4 hours, and 6 hours). The blood glucose level was measured using Glucose Test Wako (Wako Pure Chemical Industries, Ltd.). The GLP-1 peptide complexes from Examples 1, 6, 8, 9, 10, 14 and 21 were used as the GLP-1 peptide complex of the present invention, and the native GLP-1 from Comparative Example 1 and the 8S-GLP-1 from Comparative Example 2 were used as control.

2. Results and Discussion

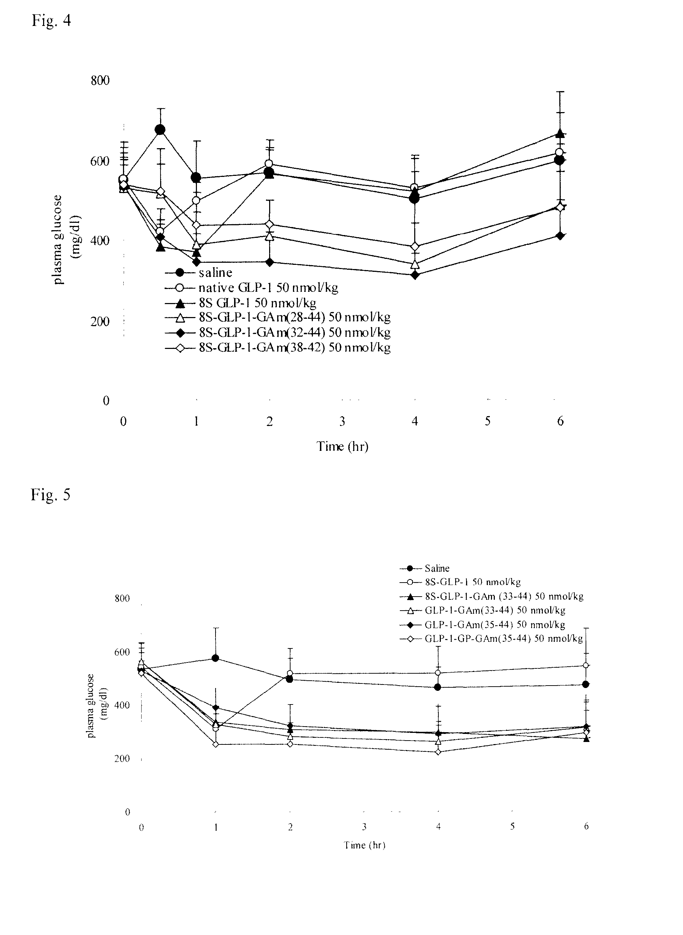

Blood glucose-lowering effect with time of each GLP-1 peptide complex after the subcutaneous administration are shown in Fig. 4 and Fig. 5. The glucose-lowering effect of the native GLP-1 (Comparative Example 1) and the 8S-GLP-1 (Comparative Example 2) disappeared one hour and two hours after the administration, respectively. On the other hand, it was confirmed that the glucose-lowering effect of the GLP-1 peptide complex in which the partial peptide of the GA module of the present invention was added reached the maximum effect one hour after the administration, and the effect was maintained over 6 hours. These results show that the GLP-1 peptide complex in which the partial peptide of the GA module of the present invention was added had remarkably improved sustainability of the blood glucose-lowering effect.
It became apparent from these results that the addition of the partial peptide of the GA module of the present invention to GLP-1 enabled to maintain the sufficient blood glucose-lowering intensity and exhibit the remarkably sustained blood glucose-lowering effect. It could be believed that the addition of the partial peptide of the GA module of the present invention to GLP-1 or another bioactive substance was useful for solving problems in medical applications of the bioactive substance, such as rapid disappearance *in vivo.*

II Synthesis of VIP Peptide Complex

VIP was selected as a bioactive substance having low biostability other than the GLP-1, and was inspected.

The partial peptide of the GA module of the present invention was added to the C-terminal of VIP to form a VIP peptide complex. The VIP peptide complex was synthesized in a solid phase synthesis, it was purified through an HPLC, and then the synthesized product was confirmed by a mass spectrometry. $-NH_2$ shows that the C-terminal is amidated.

Example 25. $VIP(1-28)-GAm(37-44)-NH_2$ SEQ ID NO: 60 (GAm: SEQ ID NO: 4)

Example 26. $VIP(1-28)-GAm(32-44)-NH_2$ SEQ ID NO: 61 (GAm: SEQ ID NO: 9)

Example 27. $VIP(1-28)-GAm(28-44)-NH_2$ SEQ ID NO: 62 (GAm: SEQ ID NO: 13)

Comparative Example 14. $VIP(I-28)-NH_2$ SEQ ID NO: 59

\<Experimental Example 4\> Evaluation of Plasma Stability of VIP Peptide Complex

The plasma stability was evaluated *in vitro,* in order to inspect the biostability of the VIP peptide complex.

1. Procedure

SD rats were anesthetized with diethyl ether, whole blood was collected, and a plasma fraction was separated therefrom. The VIP or VIP-peptide complex was added to the plasma so that the final concentration thereof was 0.5 $\mu$mol/L, and the mixture was incubated at 37°C A sample was recovered after 0, 8 or 48 hours, and a remaining rate was evaluated by an HPLC/MS measurement of an amount of the VIP or VIP peptide complex. The remaining rate of the VIP or VIP peptide complex was evaluated by calculating a rate of the VIP or VIP peptide complex remaining in the plasma recovered after 8 hours or 48 hours relative to an HPLC/MS measurement result thereof in the sample recovered at 0 hour, which was assumed as 100%.

2. Results and Discussion

The analysis results of the plasma stability of the VIP and VIP peptide complexes are shown in Table 7 as a remaining rate. The native VIP in which the partial peptide of the GA module was not added (Comparative Example 14) was completely decomposed in the plasma after 8 hours, whereas the VIP peptide complexes in which the partial peptide of the GA module was added (Examples 25, 26 and 27) were not totally decomposed in the rat plasma after 8 hours, and 118%, 47%, and 35% thereof remained respectively even after 48 hours. It became apparent from these results that the VIP peptide complexes could obtain remarkably high plasma stability by the addition of the partial peptide of the GA module of the present invention, similar to the GLP-1.

[Table 7]

| Plasma Stability | | | |
|---|---|---|---|
| Number (VIP peptide complex abbreviated name) | Partial peptide sequences of GA module | Remaining rate in plasma after 8 hours (%) | Remaining rate in plasma after 48 hours (%) |
| Example 25 VIP-GAm (37-44)-NH_2 | LIDEILAA | 118 | 118 |
| Example 26 VIP-GAm (32-44)-NH_2 | EGVKALIDEILAA | 99 | 47 |
| Example 27 VIP-GAm (28-44)-NH_2 | AKTVEGVKALIDEILAA | 109 | 35 |
| Comparative Example 14 native VIP | | 0 | 0 |

\<Experimental Example 5\> Comparison in Activity Intensity of VIP Peptide Complex

A productivity of cAMP was analyzed *in vitro,* in order to evaluate the activity intensity of the VIP peptide complexes showing the plasma stability in Experimental Example 4.

### 1. Procedure

An intracellular cAMP productivity was evaluated using SUP-T1 cells which were derived from human T-cell, expressing an endogenous VIP receptor (purchased from ATCC). The SUP-T1 cells which were subjected to suspension culture for 48 or more hours were moved to a tube, and the cells were washed twice with a medium (RPMI-1640). After that, a medium including IBMX (3-isobutyl-1-methylxanthine), which was an inhibitor of a catabolic enzyme of the cAMP, was added thereto, and the mixture was incubated at 37°C for 15 minutes. After centrifugation, the supernatant was removed, and an IBMX-containing medium including the VIP peptide complex was added thereto. The reaction was performed at 37°C for 30 minutes. After the reaction, the tube was centrifuged, the supernatant was removed, and the cell lysate was added thereto. The mixture was incubated at 37°C for 30 minutes to dissolve the cells, and the productivity was evaluated by the measurement of the cAMP. The cAMP measurement was performed using an enzyme-linked immunosorbent assay (ELISA) by chemiluminescence detection.

The activities of the native VIP (Comparative Example 14) and the VIP peptide complexes (Examples 25, 26, and 27) were evaluated in a concentration of 3, 10, 30, 100, 300, 1000 or 3000 nM, and the maximum amount of the cAMP produced was calculated. The activity intensity (%) of the VIP peptide complex was calculated according to the following formula.

$$\text{Activity Intensity (\%)} = [\{(\text{the maximum amount of the cAMP produced in each VIP peptide complex})/(\text{the maximum amount of the cAMP produced in native VIP})] \times 100$$

### 2. Results and Discussion

The results of analysis of the cAMP productivity in the VIP or VIP peptide complex are shown in Table 8 as activity intensity. It was confirmed that the activity intensities of the VIP peptide complexes (Examples 25, 26 and 27) were 81%, 85% and 67%, respectively, relative to the native VIP in which the partial peptide of the GA module was not added (Comparative Example 14), and the VIP activity could be maintained even if the partial peptide of the GA module was added. It was found from these results that the addition of the partial peptide of the GA module of the present invention could be utilized as a very useful means for practically using a bioactive substance whose defect was the biostability by obtaining the remarkably high plasma stability and maintaining the bioactivity.

[Table 8]

| cAMP Productivity in SUP-T1 Cells | | |
|---|---|---|
| Number (VIP peptide complex abbreviated name) | Partial peptide sequence of GA module | Activity Intensity (%) |
| Example 25 VIP-GAm(37-44)-NH$_2$ | LIDEILAA | 81 |
| Example 26 VIP-GAm(32-44)-NH$_2$ | EGVKALIDEILAA | 85 |
| Example 27 VIP-GAm(28-44)-NH$_2$ | AKTVEGVKALIDEILAA | 67 |
| Comparative Example 14 native VIP | | 100 |

### III Synthesis of Somatostatin (SRIF) Peptide Complex

Somatostatin (SRIF) was selected as a bioactive substance having low biostability other than the GLP-1, and was inspected. The partial peptides of the GA module of the present invention were added to the C-terminal and the N-terminal of SRIF to form an SRIF peptide complex. The SRIF peptide complex was synthesized in a solid phase synthesis, it was purified through an HPLC, and then the synthesized product was confirmed by a mass spectrometry. -NH$_2$ shows that the C-terminal is amidated.

Example 28. SRIF(1-14)-GAm(28-44)-NH$_2$ SEQ ID NO: 64 (GAm: SEQ ID NO: 13)

Example 29. GAm(28-44)-SRIF(1-14)-NH$_2$ SEQ ID NO: 65 (GAm: SEQ ID NO: 13)

Comparative Example 15. SRIF(1-14)-NH$_2$ SEQ ID NO: 63

<Experimental Example 6> Evaluation of Plasma Stability of Somatostatin peptide complex

The plasma stability was evaluated *in vitro,* in order to inspect the biostability of the somatostatin peptide complex.

1. Procedure

SD rats were anesthetized with diethyl ether, whole blood was collected, and a plasma fraction was separated therefrom. The somatostatin or somatostatin peptide complex was added to the plasma so that the final concentration thereof was 0.5 $\mu$mol/L, and the mixture was incubated at 37°C. A sample was recovered after 0, 8 or 48 hours, and a remaining rate was evaluated by an HPLC/MS measurement of an amount of the somatostatin or somatostatin peptide complex. The remaining rate of the somatostatin or somatostatin peptide complex was evaluated by calculating a rate of the somatostatin or somatostatin peptide complex remaining in the plasma recovered after 8 hours or 48 hours relative to an HPLC/MS measurement result thereof in the sample recovered at 0 hour, which was assumed as 100%.

2. Results and Discussion

The analysis results of the plasma stability of the somatostatin and somatostatin peptide complexes are shown in Table 9 as a remaining rate. The native somatostatin in which the partial peptide of the GA module was not added (Comparative Example 15) was completely decomposed in the plasma after 8 hours, whereas the somatostatin peptide complexes in which the partial peptides of GA module were added to the C-terminal and the N-terminal (Examples 28 and 29) remained in the rat plasma in a rate of 56% and 80%, respectively, after 8 hours, and the adduct at the N-terminal remained in a rate of 23% even after 48 hours. It became apparent from these results that the somatostatin peptide complexes could obtain remarkably high plasma stability by the addition of the partial peptide of the GA module of the present invention, similar to the GLP-1. It was found from the above that the partial peptide of the GA module of the present invention could be applied to the short peptide of 14 residues such as the somatostatin, and to not only the C-terminal but also the N-terminal. In addition, it has been reported that the somatostatin has an intermolecular crosslinking between the cysteine residues. The results of this experiment show that the biostability of the peptide having a cyclic structure can be improved by the partial peptide of the GA module of the present invention.

[Table 9]

| Plasma Stability | | | |
|---|---|---|---|
| Number (somatostatin peptide complex abbreviated name) | Partial peptide sequence of GA module | Remaining rate in plasma after 8 hours (%) | Remaining rate in plasma after 48 hours (%) |
| Example 28 SRIF-GAm (28-44)-NH$_2$ | AKTVEGVKALIDEILAA | 56 | 0 |
| Example 29 GAm(28-44)-SRIF-NH$_2$ | AKTVEGVKALIDEILAA | 80 | 23 |
| Comparative Example 15 native SRIF | | 0 | 0 |

<Experimental Example 7> Comparison in Activity Intensity of Somatostatin Peptide Complex

Suppressibility of insulin secretion was analyzed *in vitro,* in order to evaluate the activity intensity of the somatostatin peptide complex exhibiting the plasma stability in Experimental Example 6.

1. Procedure

The suppressibility of insulin secretion was evaluated using MIN 6 (obtained from Mr. Junichi Miyazaki) which was an insulinoma cell derived from a mouse, expressing an endogenous SSTR 5. MIN 6 was seeded on a multi-plate, which was cultivated at 37°C for 48 hours. After that, the culture was washed twice with a KRH buffer solution including 2 mM glucose solution (including NaCl 119 mM, KCl 4.74 mM, CaCl$_2$ 2.54 mM, MgCl$_2$ 1.19 mM, KH$_2$PO$_4$ 1.19 mM, 10mM HEPES buffer solution pH7.3, and 0.1% BSA), and then it was incubated at 37°C for one hour. After aspiration and removal of the solution above, a 20 mM glucose-containing KRH buffer solution including the somatostatin peptide complex was added thereto, and the resulting mixture was incubated at 37°C for one hour. A supernatant thereof was recovered, and an amount of insulin secreted was measured. The measurement of the insulin was performed in an enzyme-linked immunosorbent assay (ELISA), and an absorbance was measured at 450 nm (a sub-wavelength: 620 nm) after the addition of a substrate.

In this experiment, the activity evaluations of the native SRIF (Comparative Example 15) and the GAm (28-44)-SRIF-NH2(Example 29) were performed in a concentration of 1, 3, 10, 30, 100, 300, and 1000 nM, and the maximum suppression rate (%) of the insulin secretion was calculated. The maximum suppression rate (%) of the insulin secretion in the somatostatin peptide complex was calculated according to the following formula.

$$\text{Maximum Suppression Rate (\%) of Insulin Secretion (\%)} = [\{(\text{the maximum value of the suppression of the insulin secretion in the SRIF peptide complex})/(\text{the maximum value of the suppression of the insulin secretion in the native SRIF})] \times 100$$

2. Results and Discussion

The analysis results of the suppression rate of the insulin secretion in the somatostatin peptide complex are shown in Table 10 as an activity intensity. It was confirmed that the suppression rate of the insulin secretion in the somatostatin peptide complex (Example 29) was 90% relative to that in the native somatostatin in which the partial peptide of the GA module was not added (Comparative Example 15), and the somatostatin activity could be maintained even if the partial peptide of the GA module was added. It was found from these results that the partial peptide of the GA module of the invention enabled the bioactive substance to obtain remarkably high plasma stability and to maintain the bioactivity by adding it to the bioactive substance, and could be utilized as a very useful means for practically using the bioactive substance whose defect was the biostability.

[Table 10]

| Suppressibility of Insulin Secretion in MIN 6 Cells | | |
|---|---|---|
| Number (somatostatin peptide complex abbreviated name) | Partial peptide sequence of GA module | Suppression rate of insulin secretion (%) |
| Example 29 GAm(28-44)-SRIF-NH$_2$ | AKTVEGVKALIDEILAA | 90 |
| Comparative Example 15 native SRIF | | 100 |

IV Synthesis of Amylin Complex

Amylin was selected as a bioactive substance having low biostability other than the GLP-1, and was inspected. The partial peptide of the GA module of the present invention was added to the N-terminal of the amylin to form an amylin peptide complex. The amylin peptide complex was synthesized in a solid phase synthesis, it was purified through an HPLC, and then the synthesized product was confirmed by a mass spectrometry. -NH$_2$ shows that the C-terminal is amidated.
Example 30. GAm(28-44)-Amylin(1-37)-NH$_2$ SEQ ID NO: 67 (GAm: SEQ ID NO: 13)
Comparative Example 16. Amylin(1-37)-NH$_2$ SEQ ID NO: 66

<Experimental Example 8> Evaluation of Plasma Stability of Amylin Complex

The plasma stability was evaluated *in vitro,* in order to inspect the biostability of the amylin peptide complex. Evaluation was performed in a manner in which after the treatment of the amylin or amylin peptide complex with plasma for a pre-determined time, an amount of change of the amylin peptide (complex) undecomposed was measured by an HPLC/MS.

1. Procedure

SD rats were anesthetized with diethyl ether, whole blood was collected and a plasma fraction was separated therefrom. The amylin or the amylin peptide complex was added to the plasma so that the final concentration thereof was 0.5 μmol/L, and the mixture was incubated at 37°C. A sample was recovered after 0, 0.5, 2 or 8 hours, and a remaining rate was evaluated by an HPLC/MS measurement of an amount of the amylin or the amylin peptide complex. The remaining rate of the amylin or amylin peptide complex was evaluated by calculating a rate of the amylin or amylin peptide complex remaining in the plasma recovered after 0.5, 2 or 8 hours relative to the HPLC/MS measurement result thereof in the sample recovered at 0 hour, which was assumed as 100%.

2. Results and Discussion

The analysis results of the plasma stability of the amylin or amylin peptide complex are shown in Table 11 as a remaining rate. The native amylin in which the partial peptide of the GA module was not added (Comparative Example 16) was decomposed in the plasma to decrease to 15% after 2 hours, whereas the amylin peptide complex in which the partial peptide of the GA module was added to the N-terminal (Example 30) remained up to 67% after 2 hours. It became apparent from these results that the amylin peptide complex could obtain remarkably high plasma stability by the addition of the partial peptide of the GA module of the present invention, similar to the GLP-1. It was found from the above that the addition of the partial peptide of the GA module of the present invention could be utilized as a very useful means for practically using a bioactive substance whose defect was the biostability.
[Table 11]

| Plasma Stability | | | | |
|---|---|---|---|---|
| Number (amylin peptide complex abbreviated name) | Partial peptide sequence of GA module | Remaining rate in plasma after 0.5 hour (%) | Remaining rate in plasma after 2 hours (%) | Remaining rate in plasma after 8 hours (%) |
| Example 30 GAm(28-44)-Amylin-NH$_2$ | AKTVEGVKALIDEILAA | 88 | 67 | 9 |
| Comparative Example 16 native Amylin | | 43 | 15 | 0 |

V Synthesis of Ghrelin [(n-Octanoyl) Ghrelin] Peptide Complex

Ghrelin [(n-Octanoyl) Ghrelin] was selected as a bioactive substance having low biostability other than the GLP-1, and was inspected. The partial peptide of the GA module of the present invention was added to the C-terminal of the ghrelin to form a ghrelin peptide complex. The ghrelin peptide complex was synthesized in a solid phase synthesis, it was purified through an HPLC, and then the synthesized product was confirmed by a mass spectrometry. -NH$_2$ shows that the C-terminal is amidated.
Example 31. Ghrelin(1-28)-GAm(28-44)-NH$_2$ SEQ ID NO: 69 (GAm: SEQ ID NO: 13)
Comparative Example 17. Ghrelin(1-28)-NH$_2$ SEQ ID NO: 68

<Experimental Example 9> Evaluation of Plasma Stability of Ghrelin Peptide Complex

The plasma stability was evaluated *in vitro,* in order to inspect the biostability of the ghrelin peptide complex. Evaluation was performed in a manner in which after the treatment of the ghrelin or ghrelin peptide complex in plasma for a pre-determined time, an amount of change of the ghrelin or ghrelin peptide complex undecomposed was measured by an HPLC/MS.

1. Procedure

SD rats were anesthetized with diethyl ether, whole blood was collected and a plasma fraction was separated therefrom. The ghrelin or ghrelin peptide complex was added to the plasma so that the final concentration thereof was 0.5 μmol/L, and the mixture was incubated at 37°C. A sample was recovered after 0, 0.5, 2 or 8 hours, and a remaining rate was evaluated by an HPLC/MS measurement of an amount of the ghrelin or ghrelin peptide complex. The remaining rate of the ghrelin or ghrelin peptide complex was evaluated by calculating a rate of the ghrelin or ghrelin peptide complex remaining in the plasma recovered after 0.5, 2 or 8 hours relative to an HPLC/MS measurement result thereof in the sample recovered at 0 hour, which was assumed as 100%.

2. Results and Discussion

The analysis results of the plasma stability of the ghrelin or ghrelin peptide complex are shown in Table 12 as a remaining rate. The native ghrelin in which the partial peptide of the GA module was not added (Comparative Example 17) was decomposed in the plasma to decrease to 14% after 2 hours, whereas the ghrelin peptide complex in which the partial peptide of the GA module was added to the C-terminal (Example 31) remained in 49% after 2 hours. It was shown from these results that the partial peptide of the GA module of the present invention could be applied to a fatty acid-added peptide. From the above, the present invention can be utilized as a very useful means for practically using a bioactive substance whose defect was the biostability.
[Table 12]

| Plasma Stability | | | | |
|---|---|---|---|---|
| Number (ghrelin peptide complex abbreviated name) | Partial peptide sequende of GA module | Remaining rate in plasma after 0.5 hour (%) | Remaining rate in plasma after 2 hours (%) | Remaining rate in plasma after 8 hours (%) |
| Example 31 Ghrelin-GAm (28-44)-NH$_2$ | AKTVEGVKALIDEILAA | 85 | 49 | 6 |
| Comparative Example 17 native Ghrelin | | 50 | 14 | 0 |

[SEQUENCE]

PCT-GLP-1 sustained-release injection (SEQUENCE).txt

## Claims

1. A partial peptide of a GA module, comprising the amino acid sequence: Ile-Asp-Glu-Ile-Leu and having 5 to 25 amino acids.

2. The partial peptide according to claim 1, wherein the GA module is derived from a streptococcus G148.

3. The partial peptide according to claim 1, which has 5 to 17 amino acids.

4. The partial peptide according claim 1, which consists of the amino acid sequence depicted by the formula:

$$Y_{22}\text{-}Y_{23}\text{-}Y_{24}\text{-}Y_{25}\text{-}Y_{26}\text{-}Y_{27}\text{-}Y_{28}\text{-}Y_{29}\text{-}Y_{30}\text{-}Y_{31}\text{-}Y_{32}\text{-}Y_{33}\text{-}Y_{34}\text{-}Y_{35}\text{-}Y_{36}\text{-}Y_{37}\text{-Ile-Asp-Glu-Ile-Leu-}Y_{43}\text{-}$$

$$Y_{44}\text{-}Y_{45}\text{-}Y_{46},$$

wherein: $Y_{22}$ is Lys or deletion; $Y_{23}$ is Asn or deletion; $Y_{24}$ is Leu or deletion; $Y_{25}$ is Ile or deletion; $Y_{26}$ is Asn or deletion; $Y_{27}$ is Asn or deletion; $Y_{28}$ is Ala or deletion; $Y_{29}$ is Lys or deletion; $Y_{30}$ is Thr or deletion; $Y_{31}$ is Val or deletion; $Y_{32}$ is Glu or deletion; $Y_{33}$ is Gly or deletion; $Y_{34}$ is Val or deletion; $Y_{35}$ is Lys or deletion; $Y_{36}$ is Ala or deletion; $Y_{37}$ is Leu or deletion; $Y_{43}$ is Ala or deletion; $Y_{44}$ is Ala or deletion; $Y_{45}$ is Leu or deletion; and $Y_{46}$ is Pro or deletion, provided that the deletion is limited to a continuous deletion from $Y_{22}$ and/or $Y_{46}$ (including single deletion of $Y_{22}$ or $Y_{46}$, and deletions at two portions of $Y_{22}$ and $Y_{46}$ alone).

5. The partial peptide according to claim 4, which is selected from the group consisting of:

Ile-Asp-Glu-Ile-Leu (SEQ ID NO: 2);

Ile-Asp-Glu-Ile-Leu-Ala-Ala (SEQ ID NO: 3);
Leu-Ile-Asp-Glu-Ile-Leu-Ala-Ala (SEQ ID NO: 4);
Lys-Ala-Leu-Ile-Asp-Glu-Ile-Leu-Ala-Ala (SEQ ID NO: 5);
Val-Lys-Ala-Leu-Ile-Asp-Glu-Ile-Leu-Ala-Ala (SEQ ID NO: 6);
Gly-Val-Lys-Ala-Leu-Ile-Asp-Glu-Ile-Leu-Ala (SEQ ID NO: 7);
Gly-Val-Lys-Ala-Leu-Ile-Asp-Glu-Ile-Leu-Ala-Ala (SEQ ID NO: 8);
Glu-Gly-Val-Lys-Ala-Leu-Ile-Asp-Glu-Ile-Leu-Ala-Ala (SEQ ID NO: 9);
Val-Glu-Gly-Val-Lys-Ala-Leu-Ile-Asp-Glu-Ile-Leu-Ala-Ala (SEQ ID NO: 10);
Thr-Val-Glu-Gly-Val-Lys-Ala-Leu-Ile-Asp-Glu-Ile-Leu (SEQ ID NO: 11);
Ala-Lys-Thr-Val-Glu-Gly-Val-Lys-Ala-Leu-Ile-Asp-Glu-Ile-Leu (SEQ ID NO: 12);
Ala-Lys-Thr-Val-Glu-Gly-Val-Lys-Ala-Leu-Ile-Asp-Glu-Ile-Leu-Ala-Ala (SEQ ID NO: 13);

Asn-Ala-Lys-Thr-Val-Glu-Gly-Val-Lys-Ala-Leu-Ile-Asp-Glu-Ile-Leu-Ala-Ala-Leu-Pro (SEQ ID NO: 14);

Ile-Asn-Asn-Ala-Lys-Thr-Val-Glu-Gly-Val-Lys-Ala-Leu-Ile-Asp-Glu-Ile-Leu-Ala-Ala-Leu-Pro (SEQ ID NO: 15); and

Lys-Asn-Leu-Ile-Asn-Asn-Ala-Lys-Thr-Val-Glu-Gly-Val-Lys-Ala-Leu-Ile-Asp-Glu-Ile-Leu-Ala-Ala-Leu-Pro (SEQ ID NO: 16).

6. The partial peptide according to claim 1, which starts with one of the amino acid positions 22 to 38 of the GA module depicted in SEQ ID NO: 1 and ends with one of the amino acid positions 42 to 46 thereof.

7. A bioactive complex comprising the partial peptide according to any one of claims 1 to 6 and a bioactive substance bound to the partial peptide.

8. The bioactive complex according to claim 7, wherein the bioactive substance is a gastrointestinal hormone or a derivative thereof, or Exendin-4 or a derivative thereof.

9. The bioactive complex according to claim 8, wherein the gastrointestinal hormone is selected from the group consisting of GLP-1, GLP-2, GIP, VIP, somatostatin, amylin and ghrelin.

10. The bioactive complex according to claim 7, further comprising a linker through which the partial peptide and the bioactive substance are bound.

11. The bioactive complex according to claim 9, wherein the GLP-1 or the derivative thereof has GLP-1 activity and consists of the amino acid sequence depicted by the formula:

His-$X_8$-$X_9$-Gly-Thr-Phe-Thr-Ser-Asp-$X_{16}$-Ser-$X_{18}$-$X_{19}$-$X_{20}$-Glu-$X_{22}$-$X_{23}$-Ala-$X_{25}$-$X_{26}$-$X_{27}$-Phe-Ile-$X_{30}$-Trp-Leu-$X_{33}$-$X_{34}$-$X_{35}$-$X_{36}$-$X_{37}$-$X_{38}$-$X_{39}$-$X_{40}$-$X_{41}$-$X_{42}$-$X_{43}$-$X_{44}$-$X_{45}$,

wherein: $X_8$ is Ala, Gly, Ser, Thr, Leu, Ile, Val, Glu, Asp, Lys or Aib; $X_9$ is Glu, Gly, Asp or Lys; $X_{16}$ is Val, Ala, Gly, Ser, Thr, Leu, Ile, Tyr, Glu, Asp, Trp or Lys; $X_{18}$ is Ser, Ala, Arg, Gly, Thr, Leu, Ile, Val, Glu, Asp, Trp or Lys; $X_{19}$ is Tyr, Phe, Trp, Glu, Gln, Asp or Lys; $X_{20}$ is Leu, Met, Ala, Gly, Ser, Thr, Leu, Ile, Val, Glu, Asp, Trp, Tyr or Lys; $X_{22}$ is Gly, Ala, Glu, Ser, Thr, Leu, Ile, Val, Asp, Lys, Arg, Cys or Aib; $X_{23}$ is Gln, Arg, Glu, Asp, Asn or Lys; $X_{25}$ is Ala, Gly, Ser, Thr, Leu, Ile, Val, Glu, Asp or Lys; $X_{26}$ is Lys, Gln, Glu, Asp, His or Arg; $X_{27}$ is Glu, Ala, Asp, Lys or Leu; $X_{30}$ is Ala, Glu, Gly, Ser, Thr, Leu, Ile, Val, Asp, Lys, Gln, Tyr, His or Arg; $X_{33}$ is Val, Gly, Ala, Ser, Thr, Leu, Ile, Glu, Asp or Lys; $X_{34}$ is Lys, Arg, Glu, Asp, His, Ala, Gly or Asn; $X_{35}$ is Gly, Ala, Ser, Thr, Leu, Ile, Val, Glu, Asp, Lys, His, Pro or Aib; $X_{36}$ is Arg, Gly, Lys, Glu, Asp, His or deletion; $X_{37}$ is Gly, Pro, Glu, Lys, Ala, Thr, Ser, Asp, Leu, Ile, Val,

His or deletion; $X_{38}$ is Ser, Lys, Arg, Glu, Asp, His or deletion; $X_{39}$ is Ser, Arg, Lys, Glu, Asp, His or deletion; $X_{40}$ is Gly, Glu, Lys, Asp or deletion; $X_{41}$ is Ala, Lys, Phe, Trp, Tyr, Glu, Asp or deletion; $X_{42}$ is Pro, Lys, Glu, Asp or deletion; $X_{43}$ is Pro, Lys, Glu, Asp or deletion; $X_{44}$ is Pro, Lys, Glu, Asp or deletion; and $X_{45}$ is Ser, Lys, Val, Glu, Asp or deletion.

12. The bioactive complex according to claim 11, wherein the GLP-1 or the derivative thereof is selected from the group consisting of: GLP-1 (7-37); [Ser8]-GLP-1 (7-37); [Gly8]-GLP-1 (7-37); [Val8]-GLP-1 (7-37); [Glu22]-GLP-1 (7-37); [Lys22]-GLP-1 (7-37); [Val8, Glu22]-GLP-1 (7-37); [Val8, Lys22]-GLP-1 (7-37); [Gly8, Glu22]-GLP-1 (7-37); [Gly8, Lys22]-GLP-1 (7-37); [Val8, Glu30]-GLP-1 (7-37); [Gly8, Glu30]-GLP-1 (7-37); [Val8, His37]-GLP-1 (7-37); [Gly8, His37]-GLP-1 (7-37); [Arg34]-GLP-1 (7-37); [Lys18]-GLP-1 (7-37); [Gly8, Glu22, Gly36]-GLP-1 (7-37); [Aib8, Aib22]-GLP-1 (7-37); [Aib8, Aib35]-GLP-1 (7-37); [Aib8, Aib22, Aib35]-GLP-1 (7-37); [Glu22, Glu23]-GLP-1 (7-37); [Gly8, Glu22, Glu23]-GLP-1 (7-37); [Val8, Glu22, Glu23]-GLP-1 (7-37); [Val8, Glu22, Val25]-GLP-1 (7-37); [Val8, Glu22, Ile33]-GLP-1 (7-37); [Val8, Glu22, Val25, Ile33]-GLP-1 (7-37); GLP-1(7-36) type thereof in which the position 37 is deleted; and GLP-1(7-35) type thereof in which the positions 36 and 37 are deleted.

13. A method for improving biostability of a bioactive substance, comprising the step of binding the partial peptide according to any one of claims 1 to 6 to a bioactive substance.

14. The method according to the claim 13, wherein the bioactive substance is GLP-1 or a derivative thereof, and the partial peptide is bound to a C-terminal of the GLP-1 or the derivative thereof.

Fig. 1

Fig. 2

Fig. 3

Fig. 4

Fig. 5

**INTERNATIONAL SEARCH REPORT**

| International application No. |
|---|
| PCT/JP2011/060446 |

A. CLASSIFICATION OF SUBJECT MATTER
*C07K7/06*(2006.01)i, *C07K7/08*(2006.01)i, *C07K14/575*(2006.01)i, *C07K19/00*(2006.01)i, *A61K38/22*(2006.01)n, *A61P5/00*(2006.01)n

According to International Patent Classification (IPC) or to both national classification and IPC

B. FIELDS SEARCHED

Minimum documentation searched (classification system followed by classification symbols)
C07K1/00-19/00

Documentation searched other than minimum documentation to the extent that such documents are included in the fields searched

Electronic data base consulted during the international search (name of data base and, where practicable, search terms used)
CA/REGISTRY(STN), CAplus(STN), MEDLINE/WPIDS(STN), SwissProt/PIR/GeneSeq, JSTPlus/JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| X / A | CALVO-CALLE, J. Mauricio, et al., Human CD4+ T cell epitopes from vaccinia virus induced by vaccination or infection., PLoS pathogens, 2007, Vol.3, No.10, pages 1511-1529 | 1-2,7,10 / 3-6,8-9, 11-14 |
| X / A | GOETSCH, Liliane, et al., Identification of B- and T-cell epitopes of BB, a carrier protein derived from the G protein of Streptococcus strain G148., Clinical and diagnostic laboratory immunology, 2003, Vol.10, No.1, pages 125-132 | 1-3 / 4-14 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 July, 2011 (06.07.11) | 19 July, 2011 (19.07.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/060446

C (Continuation).   DOCUMENTS CONSIDERED TO BE RELEVANT

| Category* | Citation of document, with indication, where appropriate, of the relevant passages | Relevant to claim No. |
|---|---|---|
| Y A | WO 2009/016043 A2  (AFFIBODY AB.),<br>05 February 2009 (05.02.2009),<br>entire text<br>& AU 2008281913 A1        & CA 2694139 A1<br>& EP 2190863 A2          & CN 101765608 A<br>& IN 2010KN00460 A       & ES 2346178 T1<br>& US 2010/0273979 A1     & JP 2010-534486 A | 1-4,6-14<br>5 |
| Y A | WO 2008/005527 A2  (AMYLIN PHARMACEUTICALS,<br>INC.),<br>10 January 2008 (10.01.2008),<br>entire text<br>& EP 2044113 A2          & AU 2007269622 A1<br>& IN 2008DN10064 A       & CA 2655923 A1<br>& JP 2009-542217 A       & US 2010/0009907 A1 | 1-4,6-14<br>5 |
| A | WO 1991/001743 A1  (CEMU BIOTEKNIK AB.),<br>21 February 1991 (21.02.1991),<br>entire text<br>& SE 8902638 A          & AU 9061439 A<br>& EP 486525 A1          & JP 04-507242 A<br>& EP 486525 B1          & DE 69010206 E<br>& AU 652124 B           & SE 509359 C2<br>& US 6267964 B1         & CA 2064689 C<br>& JP 3477196 B2 | 1-14 |
| A | LINHULT, MARTIN, et al., Mutational analysis of<br>the interaction between albumin-binding domain<br>from streptococcal protein G and human serum<br>albumin., Protein science, 2002, Vol.11, No.2,<br>pages 206-213 | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1996029342 A
- WO 2009016043 A
- WO 91001743 A
- WO 2007022123 A
- WO 2008003612 A
- WO 9111457 A
- WO 9629342 A
- WO 9808871 A
- WO 9943341 A
- WO 9943708 A
- WO 9943707 A
- WO 9943706 A
- WO 9943705 A
- WO 0007617 A
- WO 08005527 A
- WO 0034331 A
- WO 02046227 A
- WO 02098348 A
- WO 03087139 A
- WO 03018516 A
- WO 05000892 A
- WO 05027978 A
- WO 06087354 A

### Non-patent literature cited in the description

- *J Med Chem,* 2000, vol. 43 (9), 1664-1669
- *Biochemistry,* 1992, vol. 31 (5), 1451-1457
- *Protein Eng Des Sel,* 2007, vol. 20 (11), 569-576
- *Protein Eng Des Sel,* 2008, vol. 21 (8), 515-527
- *Biochemistry,* 2006, vol. 45 (10), 3263-3271
- *Nature,* 1979, vol. 280 (5722), 512-514
- *Nature,* 1981, vol. 292 (5818), 55-58
- *Biochem Biophys Res Commun,* 2001, vol. 284 (3), 655-659
- *J Med Chem,* 2000, vol. 43 (23), 4370-4376